# EUROPÄISCHE PATENTSCHRIFT

(11) **EP 4 565 151 B1**
(45) Veröffentlichungstag und Bekanntmachung des Hinweises auf die Patenterteilung: **08.10.2025**
(21) Anmeldenummer: 24759100.1
(22) Anmeldetag: 20.08.2024
(51) Int. Cl.: A61B 17/29, A61B 18/14, A61B 17/00

(54) **CHIRURGISCHER PISTOLEN-HANDGRIFF SOWIE CHIRURGISCHES INSTRUMENT MIT PISTOLEN-HANDGRIFF**
SURGICAL PISTOL-GRIP HANDLE, AND SURGICAL INSTRUMENT HAVING A PISTOL-GRIP HANDLE
POIGNÉE EN FORME DE PISTOLET CHIRURGICAL, ET INSTRUMENT CHIRURGICAL AYANT UNE POIGNÉE EN FORME DE PISTOLET

(30) Priorität: 22.08.2023 DE 102023122460
(43) Veröffentlichungstag der Anmeldung: 11.06.2025
(73) Patentinhaber: Aesculap AG, 78532 Tuttlingen (DE)
(72) Erfinder: BARTHELMES, Sven, 78576 Emmingen-Liptingen (DE); ROTHWEILER, Christoph, 78166 Donaueschingen (DE)
(74) Vertreter: Winter, Brandl - Partnerschaft mbB
(86) Internationale Anmeldenummer: PCT/EP2024/073305
(87) Internationale Veröffentlichungsnummer: WO 2025/040661

(56) Entgegenhaltungen:
- US-A1- 2002 173 813
- US-A1- 2009 299 141
- US-A1- 2014 180 263
- US-B2- 10 874 403

## Beschreibung

### Technisches Gebiet

Die vorliegende Offenbarung betrifft einen chirurgischen Pistolen-Handgriff für ein chirurgisches Instrument oder eines chirurgischen Instruments, insbesondere elektrochirurgisches Instrument der minimalinvasiven Schaftbauart. Zudem betrifft die vorliegende Offenbarung ein chirurgisches Instrument mit einem solchen Pistolen-Handgriff.

Aus dem Stand der Technik sind bereits (elektro-)chirurgische Instrumente, insbesondere der minimalinvasiven Bauart, bekannt, die mittels eines insbesondere mehrteiligen, etwa aus zwei scheren-, klemmbacken-, zangen- oder pinzettenförmigen, gegeneinander/zueinander beweglichen, insbesondere verschwenkbaren Werkzeugbranchen/-elementen aufgebauten Werkzeugs ein Schneiden, Greifen, Halten und/oder Klemmen von Körpergewebe ermöglichen, um dieses durch Anlegen einer Hochfrequenzspannung monopolar oder bipolar zu koagulieren, zu veröden oder zu durchtrennen. Ein solches Instrument ist beispielsweise aus der EP 3 033 022 A1 bekannt.

Auch aus der US 2013/0304041 A1 ist ein Instrument bekannt, das einen Handgriff, einen daran angekoppelten (aus Innenschaft und Außenschaft bestehenden) Instrumentenschaft, und eine in dem Instrumentenschaft gelagerte Transmission aufweist.

Ferner ist aus der US 2014/0180263 A1 ein chirurgisches Instrument mit einem Pistolenhandgriff bekannt, bei dem der schwenkbar gelagerte Betätigungshebel über zwei Drehteile mit der Transmission gekoppelt ist.

Weiter ist aus der US 2009/0299141 A1 ein chirurgisches Instrument mit einem Pistolen-Handgriff bekannt, bei dem eine Wandlung der Translationsbewegung der Transmission über zwei Zahnräder erfolgt.

Dabei ist das Werkzeug an einem distalen (/operateurfernen bzw. patientennahen) Ende des Instruments, insbesondere an einem Instrumentenschaft des Instruments angelenkt (oder anlenkbar) und zu einer Betätigung (/Bedienung) des Werkzeugs über eine Transmission, vorzugsweise in Form einer im Inneren des Instrumentenschafts angeordneten, insbesondere längsverschieblichen Zug-/Druckstange, mit einem Handgriff an einem proximalen (/operateurnahen bzw. patientenfernen) Ende des Instruments gekoppelt. Der Handgriff weist ein der Betätigung des Werkzeugs entsprechendes Betätigungselement (etwa in Form eines Griff-/Abzughebels, einer Taste, eines Drehknopfs, eines Scherengriffs) auf, dessen (insbesondere manuelle) Betätigung/Aktuierung zu einer entsprechend umgesetzten Bewegung der Werkzeugbranchen am Einsatz-/Anwendungsort führt, wie etwa zu einer Schneide-/Greif-/Halte-/Klemmbewegung und/oder einer Dreh-/Schwenkbewegung am oder im Gewebe eines Patienten.

Elektrochirurgische Instrumente der einschlägigen Bauart verwenden in der Regel einen sogenannten Pistolen-Handgriff mit einer starren/unbeweglichen Griffschale, insbesondere in Form eines (Getriebe-)Gehäuse, das sich von distal in Richtung proximal, d.h. im Wesentlichen entlang einer Schaftachse des Instrumentenschafts, erstreckt, und eines feststehenden Griffelements, das sich in einem Winkel/quer zur distal-proximal Richtung erstreckt und an einem proximalen Endabschnitt des (Getriebe-)Gehäuses (integral/unmittelbar) an diesem ausgebildet oder (als separates, fest damit verbundenes Bauteil) daran fixiert ist. An der Griffschale ist ein insbesondere manuell betätigbarer, vorzugsweise fingerführbarer oder fingergeführter Betätigungshebel (/Abzugsbügel/-züngel) schwenkbar angelenkt, der - beispielsweise im Affengriff - durch mehrere Finger einer Greifhand eines Operateurs/Bedieners gehalten und zum Betätigen hin zur Griffschale, insbesondere zum Griffelement, manuell gezogen werden kann. Diese Zieh-/Betätigungsbewegung des Betätigungshebels/Abzugsbügels wird über ein im (Getriebe-)Gehäuse untergebrachtes Getriebe auf die Transmission innerhalb des an den Handgriff angekuppelten oder ankuppelbaren Instrumentenschafts auf das Werkzeug das Instrument übertragen, um dieses entsprechend zu bewegen/betätigen. Außerdem ist an dem Pistolen-Handgriff vorzugsweise eine Art Schalter angebracht, mittels dem das Anlegen der Hochfrequenzspannung an dem Werkzeug ausgelöst werden kann.

Wenigstens eine der beiden Werkzeugbranchen kann mit einer Elektrode oder Elektrodenreihe ausgestattet sein, über welche die Hochfrequenzspannung in das ergriffene Patientengewebe wahlweise eingeleitet werden kann. In diesem Fall läge ein elektrochirurgisches Instrument der monopolaren Bauart vor, bei der ein Patient beispielsweise auf einer Metallplatte aufliegt, über die der Hochfrequenzspannung abgeleitet wird. Alternativ hierzu können aber auch beide, sich gegenüberliegenden Werkzeugbranchen mit einer entsprechenden Elektrode oder Elektrodenreihe ausgestattet sein oder aus einem elektrisch leitfähigen Material bestehen, so dass die Hochfrequenzspannung nur in einem Spalt zwischen den Branchen anliegt. In diesem Fall läge dann ein elektrochirurgisches Instrument der bipolaren Bauart vor.

Bei bekannten Pistolen-Handgriffen ist der Betätigungshebel (/Abzugsbügel), insbesondere aufgrund einer ergonomischen und bauraumsparenden Griffgestaltung unterhalb der Schaftachse, d.h. zwischen der Schaftachse und einem proximalen Endbereich des Betätigungshebels, an dem Getriebegehäuse schwenkbar angelenkt, so dass die Ziehbewegung/Betätigungsbewegung des Betätigungshebels üblicherweise eine Translationsbewegung der Transmission in Richtung distal, d.h. eine distal gerichtete Längsbewegung der Zug-/Druckstange, hervorruft. Diese distale Translationsbewegung entspricht demnach einer Verlagerung in Schubrichtung, was jedoch Nachteile im Kraftübertragungszug mit sich bringt.

Es ist also die Aufgabe der vorliegenden Offenbarung, die Nachteile aus dem Stand der Technik zu vermeiden oder wenigstens zu verringern. Insbesondere soll ein Pistolen-Handgriff eines oder für ein chirurgisches Instrument sowie ein chirurgisches Instrument mit einem solchen Pistolen-Handgriff bereitgestellt werden, der/das besonders ergonomisch zu bedienen ist, gleichzeitig bauraumsparend aufgebaut ist und eine geeignete Kraftübertragung von dem Handgriff auf das Werkzeug ermöglicht.

Diese Aufgabe wird gelöst durch einen chirurgischen Pistolen-Handgriff eines oder für ein chirurgisches Instrument, insbesondere elektrochirurgisches Instrument mit den Merkmalen des unabhängigen Patentanspruchs und/oder durch ein chirurgisches Instrument, insbesondere elektrochirurgisches Instrument mit den Merkmalen des nebengeordneten Patentanspruchs. Vorteilhafte Ausgestaltungen und Weiterbildungen gemäß der Offenbarung sind Gegenstand der Unteransprüche.

Demnach wird die Aufgabe durch einen chirurgischen Pistolen-Handgriff (nachfolgend auch lediglich als Handgriff bezeichnet) eines oder für ein chirurgisches Instrument, insbesondere elektrochirurgisches Instrument der minimalinvasiven Schaftbauart gelöst. Der Handgriff weist ein Getriebegehäuse auf, das sich von distal in Richtung proximal (/in einer distal-proximal-Richtung, die im Wesentlichen einer Richtung entlang einer Schaftachse eines an den Handgriff angekoppelten oder ankoppelbaren Instrumentenschafts bzw. einer Schaftbaugruppe entspricht) erstreckt. Zudem weist der Handgriff ein feststehendes Griffelement (/Griffteil) auf, das sich in einem Winkel, d.h. quer, zur distal-proximal-Richtung, erstreckt und an einem proximalen Endabschnitt des Getriebegehäuses (fest daran) fixiert ist oder insbesondere an dem Getriebegehäuse ausgebildet ist, d.h. integral mit dem Getriebegehäuse ausgebildet/verbunden ist. Ferner weist der Handgriff einen an dem Getriebegehäuse schwenkbar angelenkten, insbesondere manuell betätigbaren, vorzugsweise fingerführbaren Betätigungshebel (/Abzugshebel) auf, durch den eine Betätigungskraft (durch den Operateur) aufgebracht werden kann. Weiter weist der Handgriff ein im Getriebegehäuse untergebrachtes/aufgenommenes Getriebe auf, das ausgebildet ist, um eine (durch die manuelle Betätigung hervorgerufene) Schwenkbewegung des Betätigungshebels in eine Translationsbewegung, vorzugsweise Längsbewegung einer Transmission, vorzugsweise einer innerhalb eines an den Pistolen-Handgriff distal angekuppelten/angekoppelten oder ankuppelbaren/ankoppelbaren Instrumentenschafts gelagerten Zug-/Druckstange zu übertragen. Dabei ist das Getriebe so ausgebildet ist, dass eine Schwenkbewegung des Betätigungshebels hin zum Griffelement, d.h. eine Ziehbewegung/Betätigungsbewegung des Betätigungshebels, in eine Translationsbewegung der Transmission in Richtung proximal, insbesondere eine proximal gerichtete Längsbewegung der Zug-/Druckstange, d.h. eine Zugbewegung der Zug-/Druckstange, transformiert wird. Dies hat zur Folge, dass die Schwenkbewegung des Betätigungshebels hin zum Griffelement, d.h. in einer Betätigungsrichtung, die Längsbewegung der Zug-/Druckstange in die Zugrichtung und die Schwenkbewegung des Betätigungshebels weg vom Griffelement, d.h. in einer Rückstellrichtung, die Längsbewegung der Zug-/Druckstange in die Schubrichtung hervorruft (/erzwingt/aktuiert).

Dabei bildet das Getriebe einen Kraftübertragungszug aufweisend ein erstes, mit dem Betätigungshebel in Wirkeingriff stehendes (/betätigungshebelseitiges und - gekoppeltes) Drehteil und ein (transmissionsseitiges und -gekoppeltes) zweites Drehteil, das mit dem ersten Drehteil unter Drehrichtungsumkehr in Wirkeingriff, vorzugsweise Verzahnungseingriff steht und das einen Kopplungsabschnitt für die Transmission hat. Mit anderen Worten ist das Getriebe zweiteilig oder mehrteilig (als zweiteiliger oder mehrteiliger Kraftübertragungszug) ausgebildet, so dass eine mit der Schwenkbewegung des Betätigungshebels gekoppelte Rotation des ersten Drehteils und eine mit der Translationsbewegung der Transmission gekoppelte (oder koppelbare) Rotation des zweiten Drehteils aufgrund des Wirkeingriffs zwischen dem ersten Drehteil und dem zweiten Drehteil in unterschiedliche Drehrichtungen erfolgt. Dazu stehen das erste Drehteil und das zweite Drehteil insbesondere miteinander in Verzahnungseingriff bzw. miteinander kämmende Zahne aufweisen. Die Verzahnung kann beispielsweise als eine Evolventenverzahnung ausgebildet sein.

Zudem weist das Getriebe einen an dem zweiten Drehteil angelenkten Führungsdorn auf, der in dem Getriebegehäuse aufgenommen ist. Der Führungsdorn kann insbesondere parallel zu der Translationsachse/Schaftachse längsgeführt aufgenommen sein. Vorzugsweise kann der Führungsdorn federvorgespannt aufgenommen sein. So kann eine Verlagerung des zweiten Drehteils zusätzlich geführt werden.

Ein Kern der vorliegenden Offenbarung besteht folglich darin, dass das Betätigen des Handgriffs, in Form der Schwenkbewegung des Betätigungshebels hin zum Griffelement, über einen solchen Kraftübertragungszug des Getriebes mit der Translationsbewegung/Längsbewegung der Transmission (/Zug-/Druckstange) angebunden ist, dass die Transmission in Zugrichtung (und nicht in Schubrichtung) verlagert wird, während durch das Rückstellen des Handgriffs, in Form der Schwenkbewegung des Betätigungshebels weg vom Griffelement, die Transmission in Schubrichtung (und nicht in Zugrichtung) verlagert wird. Das heißt, dass die ziehende Verlagerung der Transmission (/Zug-/Druckstange) mit einer primären Schwenkbewegung, nämlich eine durch Zusammendrücken der Hand aktuierbaren Schwenkbewegung, gekoppelt (oder koppelbar) ist.

Gemäß einer bevorzugten Ausführungsform kann das Getriebe ein Übersetzungsverhältnis von 1:1 aufweisen. Insbesondere können die Zähne des ersten Drehteils und des zweiten Drehteils auf demselben Durchmesser angeordnet sein. Alternativ könnten die Zähne des ersten Drehteils und des zweiten Drehteils auf unterschiedlichen Durchmessern angeordnet sein, um eine Untersetzung oder eine Übersetzung zu realisieren.

Gemäß einer bevorzugten Ausführungsform kann der Betätigungshebel um eine Betätigungsschwenkachse schwenkbar an dem Getriebegehäuse gelagert sein. Dabei kann die Betätigungsschwenkachse zwischen einem proximalen Endbereich des Betätigungshebels zum Aufbringen einer Betätigungskraft, d.h. eine Betätigungskraft-Angriffsstelle, und einer Translationsachse der Translationsbewegung der Transmission, insbesondere einer Längsachse der Zug-/Druckstange angeordnet sein. Das heißt, dass die Betätigungsschwenkachse bei Benutzung des Instruments/Handgriffs "unterhalb" der Translationsachse/Schaftachse angeordnet ist. Insbesondere bei einer solchen Achsordnung ist eine Drehrichtungsumkehr über das Getriebe erforderlich, um die Zugbewegung der Transmission/Zug-/Druckstange hervorrufen zu können, da ansonsten bei einer "einfachen" bzw. direkten Kopplung eines distalen Endbereichs des Betätigungshebels mit der Transmission eine Schubbewegung hervorgerufen würde.

Gemäß einer bevorzugten Ausführungsform können das erste Drehteil und das zweite Drehteil miteinander in Wirkeingriff, vorzugsweise Verzahnungseingriff stehende, umfangsseitig (nur) abschnittsweise ausgebildete Zähne aufweisen. Das heißt, dass die Zähne des ersten Drehteils und/oder zweiten Drehteils nicht über den gesamten Umfang ausgebildet sind. So kann das Getriebe besonders bauraumsparend ausgebildet werden.

Gemäß einer Weiterbildung der bevorzugten Ausführungsform kann eine Anzahl der Zähne des ersten Drehteils und/oder des zweiten Drehteils, d.h. eine Dimensionierung der umfangsseitig abschnittsweisen Ausbildung, in Abhängigkeit eines (maximalen) Hubs der Translationsbewegung der Transmission festgelegt ist. Das heißt, dass die Zähne nur in einem so großen Umfang ausgebildet sind, wie es erforderlich ist, um den Hub der Transmission zu übertragen. Insbesondere können das erste Drehteil und/oder das zweite Drehteil zwei bis fünf Zähne, vorzugsweise zwei, drei oder vier Zähne, aufweisen.

Gemäß einer Weiterbildung der bevorzugten Ausführungsform kann der Kopplungsabschnitt des zweiten Drehteils als eine axial hinterschnittene Ausnehmung zur axial hinterschneidend eingreifenden Aufnahme der Transmission ausgebildet sein, um eine Drehung des zweiten Drehteils mit der Translationsbewegung zu koppeln. Dazu kann die Transmission/Zug-/Druckstange an ihrem proximalen Ende (Endbereich) eine, insbesondere in Form eines Kugeldruckstücks ausgebildete, radiale (gegenüber einem axial angrenzenden Bereich vergrößerte) Verdickung aufweisen, die (im gekoppelten Zustand des Instrumentenschafts bzw. der Transmission bzw. der Schaftbaugruppe) in die Ausnehmung des zweiten Drehteils axial hinterschneidend eingreift. Dies hat den Vorteil, dass ein besonders kompakter und schlanker Kraftübertragungszug zur Akutierung der Translationsbewegung (und damit der Werkzeugbetätigung) realisiert werden kann.

Gemäß einer bevorzugten Ausführungsform kann das Getriebegehäuse einen Außengriff und einen fest mit dem Außengriff verbundenen, (beispielsweise angeschraubten) von dem Außengriff außenseitig abgedeckten Innengriff aufweisen, an dem das zweite Drehteil drehbar angelenkt ist. Dies hat den Vorteil, dass die Anlenkung zur Umgebung hin abgedeckt und damit geschützt ist, was insbesondere hinsichtlich Sterilisation Vorteile hat.

Gemäß einer Weiterbildung der bevorzugten Ausführungsform kann eine Federvorspannung des Führungsdorns der Schwenkbewegung des Betätigungshebels hin zum Griffelement entgegenwirken. Das heißt, dass der Betätigungshebel entgegen der Federvorspannung betätigbar ist. Dies hat den Vorteil, dass ein Kraftaufwand beim Betätigen wohl dosiert aufgebracht werden kann und das Betätigen des Werkzeugs präzise und kontrolliert erfolgen kann.

Gemäß einer bevorzugten Ausführungsform kann das feststehende Griffelement proximal zu dem Betätigungshebel angeordnet sein. So ergibt sich eine ergonomische Betätigung.

Gemäß einer bevorzugten Ausführungsform kann das feststehende Griffelement grifföffnungsfrei, insbesondere ohne eine Fingeraufnahmeöffnung und/oder ohne eine Daumenaufnahmeöffnung, ausgebildet sein. Das heißt, dass das feststehende Griffelement eingerichtet ist, von einer Hand(fläche) außenseitig umgriffen zu werden. Das heißt, dass der Handgriff nicht scherenartig bzw. ohne Nutzung des Daumens betätigbar ist.

Die Aufgabe der Offenbarung wird auch durch ein chirurgisches Instrument, insbesondere elektrochirurgisches Instrument der minimalinvasiven Schaftbauart, mit einem beschriebenen Pistolen-Handgriff gelöst.

Gemäß einer bevorzugten Ausführungsform kann das Instrument einen Instrumentenschaft aufweisen, der distal an dem Pistolen-Handgriff angekuppelt oder ankuppelbar ist. Dabei kann der Instrumentenschaft in gekoppeltem Zustand als Anschlag für die Schwenkbewegung des Betätigungshebels dienen. Das heißt, dass der Instrumentenschaft im gekoppelten Zustand als Aufschwenkbegrenzung für den Betätigungshebel, sowie als Translationsbegrenzung für die Transmission bzw. Längsbegrenzung für die Zug-/Druckstange, und damit als Wegbegrenzung für das Werkzeug dient. Das heißt auch, dass in einem entkoppelten Zustand des Instrumentenschafts die Anschlagsfunktion für die Schwenkbewegung des Betätigungshebels sowie die Translationsbegrenzung für die Transmission bzw. die Längsbegrenzung für die Zug-/Druckstange, und damit die Wegbegrenzung für das Werkzeug entfällt, so dass die beweglich geführten oder angelenkten Bestandteile des Kraftübertragungszugs des Getriebes bzw. des Instruments sich frei (bezüglich ihres Freiheitsgrads/ihrer aufgrund einer entsprechenden Lagerung/Aufnahme bzw. Anbringung möglichen Bewegung) bewegen können.

Kurzbeschreibung der Figuren
Fig. 1 zeigt eine perspektivische Ansicht eines Instruments gemäß der vorliegenden Offenbarung,
Fig. 2 zeigt eine perspektivische Ansicht eines distalen Teils des Instruments,
Fig. 3 zeigt eine vergrößerte perspektivische Ansicht eines proximalen Teils des Instruments,
Fign. 4 bis 11 zeigen eine Ausbildung bzw. Aufnahme eines Isolationsmantels des Instruments;
Fig. 12 zeigt eine Längsschnittdarstellung eines Handgriffs des Instruments;
Fign. 13 und 14 zeigen Längsschnittdarstellungen des Handgriffs in einer vollständig geöffneten bzw. geschlossenen Stellung (bzw. betätigten und unbetätigten Stellung) eines Werkzeugs des Instruments;
Fign. 15 und 16 zeigen eine Überlastsicherungsfunktion des Instruments;
Fig. 17 zeigt eine Längsschnittdarstellung des Handgriffs des Instruments in einer Ladeposition;
Fig. 18 zeigt eine Längsschnittdarstellung eines distalen Endbereichs des Instruments; und
Fig. 19 zeigt eine Querschnittsdarstellung im Bereich eines Demontageknopfs des Instruments.

### Beschreibung von bevorzugten Ausführungsformen

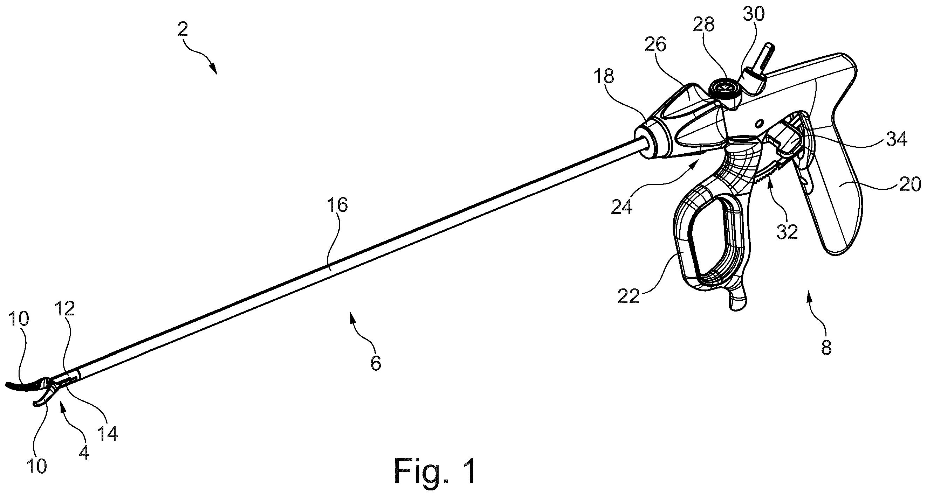

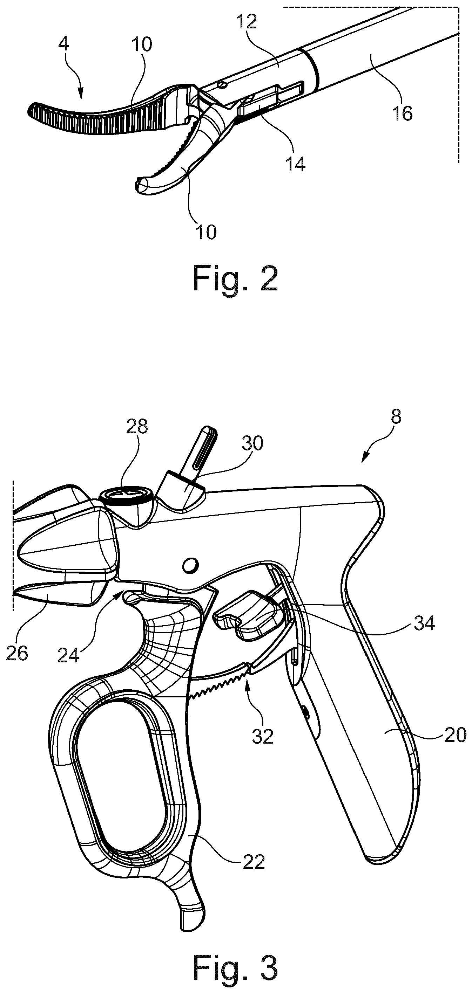

Fig. 1 zeigt eine perspektivische Ansicht eines chirurgischen Instruments 2 gemäß der vorliegenden Offenbarung. Fign. 2 und 3 zeigen vergrößerte perspektivische Ansichten eines distalen Teils bzw. proximalen Teils des Instruments 2. Das Instrument 2 ist insbesondere als ein elektrochirurgisches Instrument ausgebildet und zum Einsatz in der minimalinvasiven Chirurgie bzw. Endoskopie, insbesondere Laparoskopie vorgesehen. Das Instrument 2 ist insbesondere als ein Instrument 2 der minimalinvasiven Schaftbauart ausgebildet.

Das Instrument 2 weist ein distal angeordnetes Werkzeug 4, eine proximal zu dem Werkzeug 4 angeordnete Schaftbaugruppe 6 und einen proximal (zu dem Werkzeug 4 und der Schaftbaugruppe 6) angeordneten Handgriff 8 auf. Das heißt, dass das Werkzeug 4 an einem distalen Ende (/Arbeitsende) der Schaftbaugruppe 6 ankoppelbar oder angekoppelt ist und ein proximales Ende (/Betätigungsende) der Schaftbaugruppe 6 an dem Handgriff 8 distal ankoppelbar/ankuppelbar oder angekoppelt/angekuppelt ist. Dabei sind proximal und distal auf einen Operateur (/Bediener/Betätiger/Benutzer) des Instruments 2 bezogen definiert.

Das Instrument 2 weist das (distal angeordnete) Werkzeug 4 auf. Das Werkzeug 4 ist insbesondere mehrteilig aufgebaut und kann beispielsweise aus zwei schere-, klemmbacken-, zangen- oder pinzettenförmigen, gegeneinander/zueinander beweglichen, insbesondere zueinander verschwenkbaren Werkzeugbranchen (/elementen) 10 aufgebaut sein. Bei einem Betätigen des Werkzeugs 4 verschwenken die Werkzeugbranchen 10 relativ zueinander, wodurch sie sich öffnen bzw. schließen. Das Werkzeug 4 kann bzw. die Werkzeugbranchen 10 können zum Schneiden, Greifen, Halten und/oder Klemmen von Körpergewebe dienen. Die Werkzeugbranchen 10 sind insbesondere an der Schaftbaugruppe 6 um eine Werkzeugschwenkachse drehbar angelenkt, so dass zumindest eine der Werkzeugbranchen 10, vorzugsweise beide Werkzeugbranchen 10, relativ zu der Schaftbaugruppe 6 und damit auch relativ zu der jeweils anderen Werkzeugbranche 10 verschwenkt werden kann. Die Werkzeugschwenkachse ist insbesondere quer bzw. senkrecht zu einer distal-proximal-Richtung ausgerichtet. Die distal-proximal-Richtung entspricht insbesondere einer Längsachse der Schaftbaugruppe 6 (im Folgenden lediglich als eine Schaftachse bezeichnet). Das Werkzeug 4 ist insbesondere um seine Längsachse drehgekoppelt mit der Schaftbaugruppe 6 verbunden, so dass das Werkzeug 4 (als Ganzes) mit der Schaftbaugruppe 6 verdreht werden kann. Das Werkzeug 4 ist insbesondere aus einem Metall, vorzugsweise aus Stahl, ausgebildet.

Das Instrument 2 bzw. die Schaftbaugruppe 6 weist einen Instrumentenschaft (/Rohrschaft) 12 auf, dessen Längsachse (/Rohrachse) insbesondere der Schaftachse entspricht. Der Instrumentenschaft 12 kann vorzugsweise translatorisch fest und vorzugsweise um die Schaftachse drehbar aufgenommen sein. Das Werkzeug 4 kann mit dem Instrumentenschaft 12 derart gekoppelt sein, dass eine Rotation des Instrumentenschafts 12 (um die Schaftachse) eine Rotation des Werkzeugs 4 (um die Schaftachse) hervorruft (/erzwingt/aktuiert). Insbesondere können das Werkzeug 4 und der Instrumentenschaft 12, vorzugsweise direkt, um die Schaftachse drehfest miteinander verbunden sein. Der Instrumentenschaft 12 ist insbesondere aus einem Metall, vorzugsweise aus Stahl, ausgebildet.

Das Instrument 2 bzw. die Schaftbaugruppe 6 weist eine Transmission, vorzugsweise eine im Inneren/innerhalb des Instrumentenschafts 12 gelagerte (/aufgenommene/angeordnete) Zug-/Druckstange 14 auf, deren Längsachse (/Stangenachse) insbesondere der Schaftachse bzw. im Wesentlichen der distal-proximal-Richtung entspricht. Die Transmission (/die Zug-/Druckstange 14) kann translatorisch verschiebbar, vorzugsweise axial-/längsbeweglich/-verschieblich, d.h. translatorisch entlang der Schaftachse verschieblich, und vorzugsweise rotatorisch fest aufgenommen sein. Das Werkzeug 4 kann mit der Transmission (/Zug-/Druckstange 14) derart gekoppelt sein, dass eine Translationsbewegung, insbesondere Längsbewegung (entlang der Schaftachse) ein Betätigen des Werkzeugs 4, insbesondere eine Schwenkbewegung (bzw. Öffnen und Schließen) der Werkzeugbranchen 10 (um die Werkzeugschwenkachse), hervorruft (/erzwingt/aktuiert). Insbesondere können das Werkzeug 4 und die Zug-/Druckstange 14, vorzugsweise über einen Kopplungsmechanismus, miteinander verbunden sein. Das heißt, dass die Zug-/Druckstangen-Längsbewegung in eine distale Richtung/Schubrichtung das Öffnen (oder Schließen) des Werkzeugs 4 bzw. der Werkzeugbranchen 10 und in eine proximale Richtung/Zugrichtung das Schließen (oder Öffnen) des Werkzeugs 4 bzw. der Werkzeugbranchen 10 hervorruft (/erzwingt/aktuiert). Die Zug-/Druckstange 14 ist insbesondere aus einem Metall, vorzugsweise aus Stahl, ausgebildet.

Das Instrument 2 bzw. die Schaftbaugruppe 6 weist einen insbesondere auf dem Instrumentenschaft 12 angeordneten Isolationsmantel 16 auf, dessen Längsachse (/Mantelachse) insbesondere der Schaftachse entspricht. Der Isolationsmantel 16 kann, insbesondere relativ zu dem Instrumentenschaft 12 und/oder der Zug-/Druckstange 14, axial-/längsbeweglich/-verschieblich, d.h. translatorisch entlang der Schaftachse verschieblich, und vorzugsweise (frei) drehbar aufgenommen sein. Der Isolationsmantel 16 ist hohl, vorzugsweise rohrförmig, ausgebildet und dient zur (radial) außenseitigen elektrischen Isolierung des Instrumentenschafts 12 und/oder der Zug-/Druckstange 14, insbesondere zwischen einem distalen Endbereich und einem proximalen Endbereich des Instrumentenschafts 12 und/oder der Zug-/Druckstange 14. Der Isolationsmantel 16 ist aus einem zum Instrumentenschaft 12 unterschiedliche Material, insbesondere aus einem Kunststoff, vorzugsweise aus PEEK, ausgebildet. Eine Ausbildung bzw. Aufnahme des Isolationsmantels 16 wird untenstehend näher beschrieben.

Das Instrument 2 bzw. die Schaftbaugruppe 6 weist eine insbesondere auf dem Instrumentenschaft 12 angeordnete, vorzugsweise im Querschnitt ringförmige Kappe 18 auf. Durch eine (zentrale) Öffnung der Kappe 18 können der Instrumentenschaft 12, die Zug-/Druckstange 14 und/oder der Isolationsmantel 16 axial hindurchgeführt sein. Die Kappe 18 kann, insbesondere relativ zu dem Instrumentenschaft 12, translatorisch fest und vorzugsweise rotatorisch fest aufgenommen sein. Die Kappe 18 dient als Axialanschlag der Schaftbaugruppe 6 an dem Handgriff 8.

Das Instrument 2 weist den (proximal angeordneten) Handgriff 8 auf. Der Handgriff 8 ist insbesondere nach Art eines Pistolengriffs bzw. als ein Pistolen-Handgriff ausgebildet. Der Handgriff 8 weist ein Getriebegehäuse 20 auf, das sich insbesondere von distal in Richtung proximal, d.h. in der distal-proximal-Richtung bzw. im Wesentlichen parallel zur/entlang der Schaftachse, erstreckt.

Zudem weist der Handgriff 8 ein feststehendes Griffelement (/Griffteil) 21 auf. Das Griffelement 21 erstreckt sich insbesondere in einem Winkel, d.h. quer, zur distal-proximal-Richtung. Das Griffelement 21 kann an einem proximalen Endabschnitt des Getriebegehäuses 20 fixiert sein oder insbesondere an dem Getriebegehäuse 20 ausgebildet sein, d.h. integral mit dem Getriebegehäuse 20 verbunden sein. Insbesondere sind das Getriebegehäuse 20 und das Griffelement 21 fest miteinander verbunden.

Weiter weist der Handgriff 8 einen an dem Getriebegehäuse 20 angelenkten, insbesondere fingergeführten bzw. fingerführbaren Betätigungshebel 22 auf. Der Betätigungshebel 22 ist insbesondere manuell betätigbar und weist eine Angriffsstelle zum Aufbringen einer Betätigungskraft (durch den Operateur) aus. Der Betätigungshebel 22 kann eine, vorzugsweise geschlossene bzw. im Wesentlichen ringförmige, Schlaufe zur Aufnahme von Fingern (vorzugsweise nicht eines Daumens) des Operateurs aufweisen, welche die Betätigungskraft-Angriffsstelle bildet. Der Betätigungshebel 22 kann schwenkbar aufgenommen sein. Der Betätigungshebel 22 ist insbesondere an dem Getriebegehäuse 20 um eine Betätigungshebelschwenkachse drehbar angelenkt, so dass der Betätigungshebel 22 relativ zu dem Getriebegehäuse 20, d.h. zu dem Griffelement 21 hin oder von dem Griffelement 21 weg, verschwenkt werden kann. Die Betätigungshebelschwenkachse ist insbesondere quer bzw. senkrecht zu der Schaftachse, d.h. der distal-proximal-Richtung. Durch eine manuelle Betätigung des Betätigungshebels 22, d.h. durch ein Aufbringen der Betätigungskraft an der Betätigungskraft-Angriffsstelle, insbesondere der Schlaufe, wird eine Schwenkbewegung des Betätigungshebels 22 (relativ zu dem Getriebegehäuse 20) hervorgerufen (/erzwungen/aktuiert). Die Schwenkbewegung des Betätigungshebels 22 zu dem Griffelement 21 hin, aktuiert etwa durch Schließen einer Hand des Operateurs/Zusammendrücken von Betätigungshebel 22 und Griffelement 21, wird im Folgenden lediglich als Verschwenken/Schwenkbewegung in eine Betätigungsrichtung bzw. Betätigen des Betätigungshebels 22 bezeichnet. Die Schwenkbewegung des Betätigungshebels 22 von dem Griffelement 21 weg, aktuiert etwa durch Öffnen einer Hand des Operateurs/Auseinanderdrücken von Betätigungshebel 22 und Griffelement 21, wird im Folgenden lediglich als Verschwenken/Schwenkbewegung in eine Rückstellrichtung bzw. Rückstellen des Betätigungshebels 22 bezeichnet.

Das Instrument 2 bzw. der Handgriff 8 weist ein Getriebe 24 auf, das die (durch die manuelle Betätigung aktuierte) Schwenkbewegung des Betätigungshebels 22 in eine Translationsbewegung der Transmission, insbesondere in eine Längsbewegung der Zug-/Druckstange 14 überträgt. Das heißt, dass das Getriebe 24 die Schwenkbewegung des Betätigungshebels 22 mit der Längsbewegung der Zug-/Druckstange 14 (und damit (indirekt) mit dem Betätigen des Werkzeugs 4 bzw. dem Öffnen und dem Schließen der Werkzeugbranchen 10) koppelt. Mit anderen Worten aktuiert das Betätigen des Betätigungshebels 22 die Längsbewegung der Zug-/Druckstange 14 in die Schubrichtung (oder in die Zugrichtung) und das Rückstellen des Betätigungshebels 22 die Längsbewegung der Zug-/Druckstange 14 in die Zugrichtung (oder in die Schubrichtung) (welche wiederum das Betätigen (bzw. Öffnen oder Schließen) des Werkzeugs 4 hervorruft). Das Getriebe 24 kann vorzugsweise größtenteils oder vollständig innerhalb des Getriebegehäuses 20 angeordnet sein bzw. von dem Getriebegehäuse 20 nach außen hin abgedeckt sein. Eine Ausbildung des Getriebes 24 wird untenstehend näher beschrieben.

Das Instrument 2 bzw. der Handgriff 8 weist einen, insbesondere an einem distalen Ende des Handgriffs 8 angeordneten Drehstern 26 auf, dessen Längsachse (/Sternachse) insbesondere der Schaftachse entspricht. Der Drehstern 26 kann, insbesondere relativ zu dem Getriebegehäuse 20, vorzugsweise translatorisch fest und vorzugsweise um die Schaftachse drehbar aufgenommen sein. Durch eine (zentrale) Öffnung des Drehsterns 26 können der Instrumentenschaft 12, die Zug-/Druckstange 14 und/oder der Isolationsmantel 16 axial hindurchgeführt oder hindurchführbar sein. Der Drehstern 26 kann mit dem Instrumentenschaft 12 derart koppelbar oder gekoppelt sein, dass eine Rotation des Drehsterns 26 (um die Schaftachse) die Rotation des Instrumentenschafts 12 (um die Schaftachse) hervorruft (/erzwingt/aktuiert) (welche wiederum die Rotation des Werkzeugs 4 hervorruft). Insbesondere können der Drehstern 26 und der Instrumentenschaft 12, vorzugsweise direkt oder über ein mit dem Instrumentenschaft 12 (fest) verbundenes Bauteil, um die Schaftachse drehfest miteinander verbunden sein.

Das Instrument 2 bzw. der Handgriff 8 weist einen Demontageknopf 28 auf, bei dessen Betätigung (/Drücken) die Schaftbaugruppe 6 und der Handgriff 8 demontierbar sind, d.h. die Schaftbaugruppe 6 aus dem Handgriff 8 auskoppelbar ist. Eine Ausbildung des Demontageknopfs 28 wird untenstehend näher beschrieben.

Das Instrument 2 bzw. der Handgriff 8 weist einen Hochfrequenzanschluss, insbesondere einen HF-Pin 30 auf, durch den das Werkzeug 4, insbesondere die Werkzeugbranchen 10, mit einer Hochfrequenzspannung beaufschlagbar ist. Der **HF-**Pin 30 kann, insbesondere relativ zu dem Getriebegehäuse 20, vorzugsweise translatorisch fest und vorzugsweise rotatorisch fest aufgenommen sein. Der HF-Pin 30 kann in Kontakt mit dem Instrumentenschaft 12 und/oder der Zug-/Druckstange 14 sein oder bringbar sein, um die Hochfrequenzspannung durch das Material des Instrumentenschafts 12 und/oder der Zug-/Druckstange 14 an das Werkzeug 4 zu übertragen. Der HF-Pin 30 kann als ein bipolarer oder als ein monopolarer HF-Pin ausgebildet sein.

Der Handgriff 8 weist einen Rastmechanismus 32 auf, durch den eine Schwenkposition des Betätigungshebels 22 in vorbestimmten Rastpositionen feststellbar ist. Der Rastmechanismus 32 kann aus einem am Griffelement 21 (fest) angebrachten Rastbügel und einer am Betätigungshebel 22 (fest) angebrachten Raste aufgebaut sein.

Der Handgriff 8 weist einen Taster 34 auf, mit dem eine Beaufschlagung des Werkzeugs 4 mit der Hochfrequenzspannung ausgelöst werden kann. Alternativ kann der Taster 34 zur Entriegelung des Rastmechanismus 32 dienen.

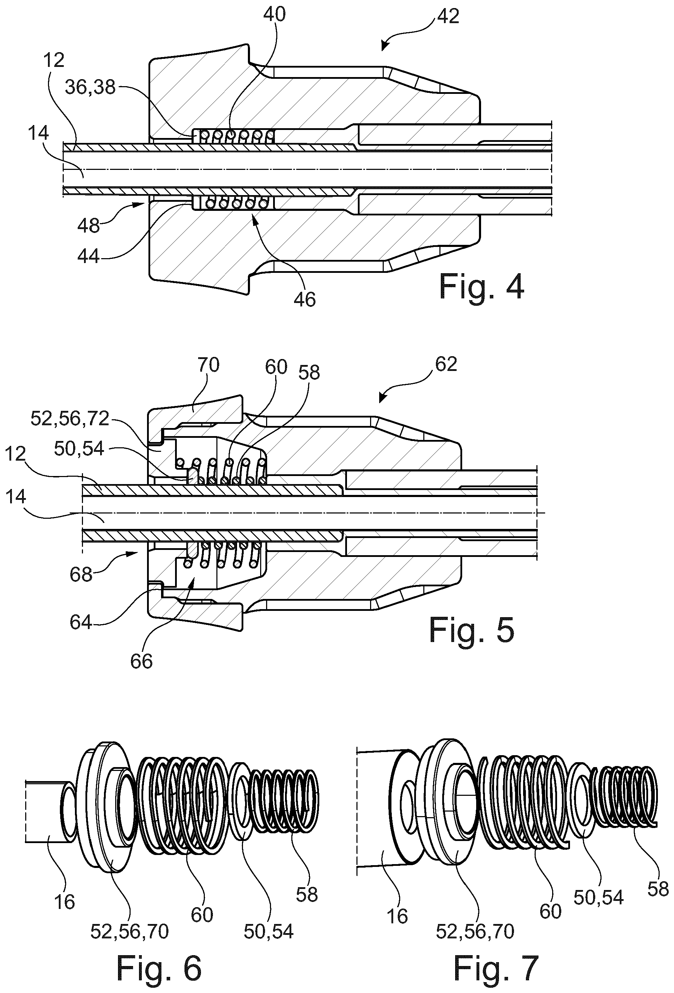

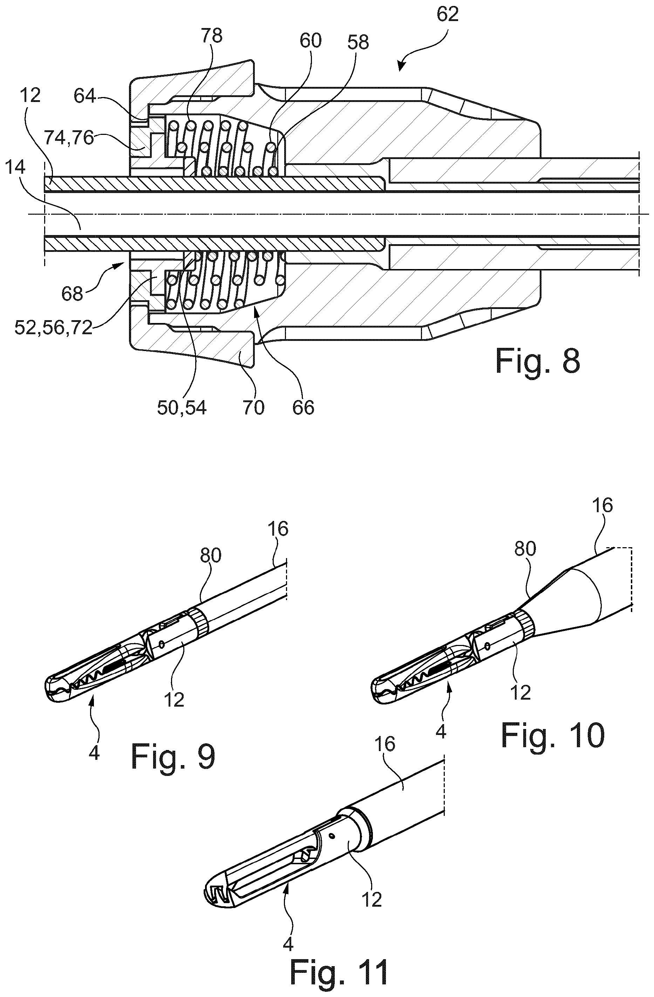

Die Ausbildung bzw. Aufnahme des Isolationsmantels 16 wird mit Bezugnahme auf Fign. 4 bis 11 beschrieben. Fig. 4 zeigt eine Längsschnittdarstellung einer proximalen Aufnahme des Isolationsmantels 16 gemäß einer ersten Ausführungsform. Fig. 5 zeigt eine Längsschnittdarstellung einer proximalen Aufnahme des Isolationsmantels 16 gemäß einer zweiten Ausführungsform. Fign. 6 und 7 zeigen Explosionsdarstellungen von einzelnen Teilen der proximalen Aufnahme des Isolationsmantels 16 gemäß der zweiten Ausführungsform. Fig. 8 zeigt eine Längsschnittdarstellung einer proximalen Aufnahme des Isolationsmantels 16 gemäß einer dritten Ausführungsform. Fign. 9 bis 11 zeigen unterschiedliche Ausführungsformen eines distalen Abschnitts des Isolationsmantels 16 und einer distalen Aufnahme des Isolationsmantels 16.

Der Isolationsmantel 16 ist, wie oben beschrieben, axial verschieblich (/frei schwimmend) auf dem Instrumentenschaft 12 aufgenommen. An dem Instrument 2 ist ein proximaler Axialanschlag 36 zur Begrenzung einer proximal gerichteten Axialbewegung (in Richtung zum Handgriff 8 hin) des Isolationsmantels 16 ausgebildet.

Gemäß einem Aspekt der Offenbarung ist der proximale Axialanschlag 36 axial verschieblich auf dem Instrumentenschaft 12 aufgenommen/angeordnet. Dabei ist der proximale Axialanschlag 36 distal/in eine distale Richtung axialvorgespannt. Das heißt, dass der proximale Axialanschlag 36 eine distal gerichtete Axialkraft auf den Isolationsmantel 16 aufbringt bzw. der proximale Axialanschlag 36 durch eine axiale Vorspannung distal/in die distale Richtung gedrückt wird.

**In** einem abgekühlten Zustand befindet sich der Isolationsmantel 16 in seiner Montageposition und liegt axial an dem proximalen Axialanschlag 36 an. Durch eine Erwärmung, etwa bei einer Sterilisation des Instruments 2 und/oder der Schaftbaugruppe 6, dehnen sich der Isolationsmantel 16 und der Instrumentenschaft 12, aufgrund ihrer unterschiedlichen Wärmeausdehnungskoeffizienten, unterschiedlich weit aus, so dass es zu einem axialen Verrutschen des Isolationsmantels 16 auf dem Instrumentenschaft 12 kommt. Der proximale Axialanschlag 36 verschiebt sich durch eine Ausdehnung des Isolationsmantels 16 in eine proximale Richtung, wodurch die axiale Vorspannung auf den proximalen Axialanschlag 36 zunimmt. Wenn der Isolationsmantel 16 wieder abkühlt und sich zusammenzieht, wird der Isolationsmantel 16 durch die axiale Vorspannung des proximalen Axialanschlag 36 in seine Montageposition zurückgeschoben.

Fig. 4 zeigt eine erste Ausführungsform der Ausbildung der proximalen Aufnahme des Isolationsmantels 16. Der proximale Axialanschlag 36 ist vorzugsweise an einer insbesondere auf dem Instrumentenschaft 12 aufgenommenen (etwa auf einen Außenumfang des Instrumentenschafts 12 aufgesteckten/aufgesetzten) ringförmigen Scheibe 38 ausgebildet. Die axiale Vorspannung des proximalen Axialanschlags 36 ist vorzugsweise durch ein insbesondere auf dem Instrumentenschaft 12 angeordnetes, axial vorspannendes Druckelement, insbesondere eine Feder 40, vorzugsweise in Form einer Schraubenfeder, realisiert. Die Feder 40 kann vorzugsweise direkt an der Scheibe 38 anliegen. Alternativ kann die Feder 40 vorzugsweise direkt an dem Isolationsmantel 16 anliegen, so dass eine axiale Stirnfläche der Feder 40 den proximalen Axialanschlag 36 ausbildet. Der proximale Axialanschlag 36 (d.h. die Scheibe 38 und/oder die Feder 40) kann vorzugsweise innerhalb eines insbesondere auf dem Instrumentenschaft 12 aufgenommenen, kapselartigen Adapters 42 angeordnet sein. Der Adapter 42 kann zur (radial) außenseitigen elektrischen Isolierung des Instrumentenschafts 12 und/oder der Zug-/Druckstange 14 dienen. Der Adapter 42 kann vorzugsweise axialfest mit dem Instrumentenschaft 12 verbunden sein.

Der Adapter 42 kann vorzugsweise einen distalen Anschlag 44 zur Begrenzung einer distal gerichteten Axialbewegung (in Richtung zum Werkzeug 4 hin) des axialverschieblichen proximalen Axialanschlags 36 (d.h. der Scheibe 38 und/oder der Feder 40) aufweisen. Dabei kann eine Axialposition des distalen Anschlags 44 vorzugsweise in Abhängigkeit eines Wärmeausdehungsverhaltens des Isolationsmantels 16 und/oder des Instrumentenschafts 12 festgelegt sein. Insbesondere kann die Axialposition so festgelegt sein, dass der Isolationsmantel 16 in dem abgekühlten Zustand an dem proximalen Axialanschlag 36 (sowie einem später beschriebenen distalen Axialanschlag 80) axial anliegt (d.h., dass sich der Isolationsmantel 16 nicht aufgrund seines Wärmeausdehungsverhaltens weiter in die distale Richtung zurückziehen soll als der proximale Axialanschlag 36 aufgrund des distalen Anschlags 44 in die distale Richtung gedrückt werden kann). Der distale Anschlag 44 kann insbesondere an einem sich radial nach innen (insbesondere weiter über einen Außenumfang des proximalen Axialanschlags 36/der Scheibe 38 hinaus nach innen) erstreckenden Abschnitt des Adapters 42 ausgebildet sein.

Der Adapter 42 kann vorzugsweise eine Aufnahmeschale 46 mit einer Einlegeöffnung zum (axialen) Einlegen (/Einschieben/Einsetzen) des proximalen Axialanschlags 36, insbesondere der Scheibe 38 und/oder der Feder 40, aufweisen. Die Einlegeöffnung kann insbesondere einen größeren Außendurchmesser als der proximale Axialanschlag 36, insbesondere als die Scheibe 38 und/oder die Feder 40, aufweisen. Die Einlegeöffnung kann vorzugsweise auf einer proximalen Seite des Adapters 42 ausgebildet sein. Die Aufnahmeschale 46 kann den distalen Anschlag 44 vorzugsweise direkt/integral ausbilden.

Der Adapter 42 kann vorzugsweise eine distale Öffnung 48 aufweisen, die insbesondere im Wesentlichen so groß ist wie ein Außendurchmesser des Isolationsmantels 16 (bzw. geringfügig größer um die Axialverschiebbarkeit des Isolationsmantels 16 zu gewährleisten), durch die der Isolationsmantel 16 (sowie der Instrumentenschaft 12 und/oder die Zug-/Druckstange 14) axial hindurchgeführt oder hindurchführbar ist. Die distale Öffnung 48 kann (direkt/integral) an einem Innenumfang (/-durchmesser) der Aufnahmeschale 46 ausgebildet sein.

Fign. 5 bis 7 zeigen eine zweite Ausführungsform der Ausbildung der proximalen Aufnahme des Isolationsmantels 16. Die proximale Aufnahme gemäß der zweiten Ausführungsform kann universell für (zwei) Isolationsmäntel 16 unterschiedlichen Durchmessers dienen. Die zweite Ausführungsform unterscheidet sich insbesondere dadurch von der ersten Ausführungsform, dass zwei proximale Axialanschläge 36 vorhanden sind. Ein erster proximaler Axialanschlag 50 dient zur Begrenzung der Axialbewegung des Isolationsmantels 16 mit einem ersten (kleineren) Durchmesser, während ein zweiter proximale Axialanschlag 52 zur Begrenzung der Axialbewegung des Isolationsmantels 16 mit einem zweiten (größeren) Durchmesser dient, wobei der erste proximale Axialanschlag 50 und der zweite proximale Axialanschlag 52 jeweils im Aufbau im Wesentlichen dem proximalen Axialanschlag 36 der ersten Ausführungsform entsprechen.

Der erste bzw. zweite proximale Axialanschlag 50, 52 ist vorzugsweise an einer insbesondere auf dem Instrumentenschaft 12 aufgenommenen (etwa auf einen Außenumfang des Instrumentenschafts 12 aufgesteckte/aufgesetzte) ringförmigen ersten Scheibe 54 bzw. zweiten Scheibe 56 ausgebildet. Die axiale Vorspannung des ersten bzw. zweiten proximalen Axialanschlags 50, 52 ist vorzugsweise durch ein insbesondere auf dem Instrumentenschaft 12 angeordnetes, axial vorspannendes Druckelement, insbesondere eine erste Feder 58 bzw. zweite Feder 60, vorzugsweise in Form einer Schraubenfeder, realisiert. Die erste bzw. zweite Feder 58, 60 kann vorzugsweise direkt an der ersten bzw. zweiten Scheibe 54, 56 anliegen. Alternativ kann die erste bzw. zweite Feder 58, 60 vorzugsweise direkt an dem ersten bzw. zweiten Isolationsmantel 16 anliegen, so dass eine axiale Stirnfläche der ersten bzw. zweiten Feder 58, 60 den ersten bzw. zweiten proximalen Axialanschlag 50, 52 ausbildet.

**In** der proximalen Aufnahme ist insbesondere immer nur ein erster oder zweiter Isolationsmantel 16 eingesetzt. Fig. 6 zeigt eine Explosionsdarstellung (von links nach rechts betrachtet) des Isolationsmantels 16 mit dem ersten (kleineren) Durchmesser, der (den zweiten proximalen Axialanschlag 52 bildenden) zweiten Scheibe 56, der zweiten Feder 60, der ersten Scheibe 54 und der ersten Feder 58. Fig. 7 zeigt eine Explosionsdarstellung (von links nach rechts betrachtet) des Isolationsmantels 16 mit dem zweiten (größeren) Durchmesser, der (den zweiten proximalen Axialanschlag 52 bildenden) zweiten Scheibe 56, der zweiten Feder 60, der ersten Scheibe 54 und der ersten Feder 58.

Vorzugsweise können die erste Feder 58 und die zweite Feder 60 unterschiedliche Federhärten aufweisen. Insbesondere kann die zweite Feder 60 eine größere Federhärte als die erste Feder aufweisen.

Vorzugsweise können die erste Feder 58 und die zweite Feder 60 radial geschachtelt/ineinander angeordnet sein. Insbesondere kann die erste Feder 58 radial innerhalb der zweiten Feder 60 angeordnet sein.

Vorzugsweise können die erste Scheibe 54 und die zweite Scheibe 56 axial aneinander anliegen. Insbesondere kann die erste Scheibe 54 proximal der zweiten Scheibe 56 angeordnet sein.

Vorzugsweise können die erste Scheibe 54 und die zweite Scheibe 56 unterschiedliche Innendurchmesser aufweisen. Insbesondere kann die distal angeordnete Scheibe 54, 56, hier die zweite Scheibe 56, einen größeren Innendurchmesser als die proximal angeordnete Scheibe 54, 56, hier die erste Scheibe 54, aufweisen, so dass der jeweilige Isolationsmantel 16 zum axialen Anliegen an der proximal angeordneten Scheibe 54, 56, hier der ersten Scheibe 54, durch die distal angeordnete Scheibe 54, 56, hier durch die zweite Scheibe 56, axial hindurchführbar ist. Alternativ kann die distal angeordnete Scheibe 54, 56 ein Durchgangsloch aufweisen, durch das der jeweilige Isolationsmantel 16 zum axialen Anliegen an der proximal angeordneten Scheibe 54, 56 hindurchführbar ist.

Der erste bzw. zweite proximale Axialanschlag 50, 52 (d.h. die erste bzw. zweite Scheibe 54, 56 und/oder die erste bzw. zweite Feder 58, 60) kann vorzugsweise innerhalb eines insbesondere auf dem Instrumentenschaft 12 aufgenommenen, kapselartigen Adapters 62 angeordnet sein. Der Adapter 62 kann zur (radial) außenseitigen elektrischen Isolierung des Instrumentenschafts 12 und/oder der Zug-/Druckstange 14 dienen. Der Adapter 62 kann vorzugsweise axialfest mit dem Instrumentenschaft 12 verbunden sein.

Der Adapter 62 kann vorzugsweise einen distalen Anschlag 64 zur Begrenzung einer distal gerichteten Axialbewegung (in Richtung zum Werkzeug 4 hin) des axialverschieblichen ersten bzw. zweiten proximalen Axialanschlags 50, 52 aufweisen. Dabei kann eine Axialposition des distalen Anschlags 64 vorzugsweise in Abhängigkeit eines Wärmeausdehungsverhaltens des Isolationsmantels 16 und/oder des Instrumentenschafts 12 festgelegt sein. Insbesondere kann die Axialposition so festgelegt sein, dass der Isolationsmantel 16 in dem abgekühlten Zustand an dem ersten bzw. zweiten proximalen Axialanschlag 50, 52 (sowie einem später beschriebenen distalen Axialanschlag 80) axial anliegt (d.h., dass sich der Isolationsmantel 16 nicht aufgrund seines Wärmeausdehungsverhaltens weiter in die distale Richtung zurückziehen soll als der erste bzw. zweite proximale Axialanschlag 50, 52 aufgrund des distalen Anschlags 64 in die distale Richtung gedrückt werden kann). Der distale Anschlag 64 kann insbesondere an einem sich radial nach innen (insbesondere weiter über einen Außenumfang des ersten bzw. zweiten proximalen Axialanschlags 50, 52 hinaus nach innen) erstreckenden Abschnitt des Adapters 62 ausgebildet sein.

Der Adapter 62 kann vorzugsweise eine Aufnahmeschale 66 mit einer Einlegeöffnung zum (axialen) Einlegen (/Einschieben/Einsetzen) des ersten bzw. zweiten proximalen Axialanschlags 50, 52, insbesondere der ersten bzw. zweiten Scheibe 54, 56 und/oder der ersten bzw. zweiten Feder 58, 60, aufweisen. Die Einlegeöffnung kann insbesondere einen größeren Außendurchmesser als der erste bzw. zweite proximale Axialanschlag 50, 52, insbesondere als die erste bzw. zweite Scheibe 54, 56 und/oder die erste bzw. zweite Feder 58, 60, aufweisen. Die Einlegeöffnung kann vorzugsweise auf einer distalen Seite des Adapters 62 ausgebildet sein.

Der Adapter 62 kann vorzugsweise eine distale Öffnung 68 aufweisen, deren Außendurchmesser insbesondere im Wesentlichen dem des Isolationsmantels 16 (bzw. geringfügig größer ist um die Axialverschiebbarkeit des Isolationsmantels 16 zu gewährleisten), durch die der Isolationsmantel 16 (sowie der Instrumentenschaft 12 und/oder die Zug-/Druckstange 14) axial hindurchgeführt oder hindurchführbar ist. Alternativ kann die distale Öffnung 68 im Wesentlichen so groß wie ein Außendurchmesser des Instrumentenschafts 12 sein, wenn der Isolationsmantel 16 zur Kontaktierung der distal angeordneten Scheibe 54, 56, hier der ersten Scheibe 54, durch das Durchgangsloch hindurchgreift.

Der Adapter 62 eine vorzugsweise separat von der Aufnahmeschale 66 ausgebildete Kappe 70 aufweisen, die von distaler Seite auf die Aufnahmeschale 66 aufsteckbar, insbesondere aufschraubbar ist. Die Kappe 70 kann den distalen Anschlag 64 vorzugsweise direkt/integral ausbilden. Der Adapter 62 kann vorzugsweise einen Axialdeckel 72 aufweisen, der die Einlegeöffnung an der Aufnahmeschale 66 verschließt und die distale Öffnung 68 an seinem Innenumfang (/-durchmesser) (direkt/integral) ausbildet. Der Axialdeckel 72 kann vorzugsweise durch die erste Scheibe 54 oder die zweite Scheibe 56 gebildet sein, insbesondere durch die proximal angeordnete Scheibe 54, 56, hier die zweite Scheibe 56. Alternativ kann die distale Öffnung 68 (direkt/integral) an einem Innenumfang (/-durchmesser) der Kappe 70 ausgebildet sein, wenn der Isolationsmantel 16 zur Kontaktierung der distal angeordneten Scheibe 54, 56, hier der ersten Scheibe 54, durch ein Durchgangsloch in der Kappe 70 hindurchgreift.

Fig. 8 zeigt eine dritte Ausführungsform der Ausbildung der proximalen Aufnahme des Isolationsmantels 16. Die proximale Aufnahme gemäß der dritten Ausführungsform kann universell für (drei) Isolationsmäntel 16 unterschiedlichen Durchmessers dienen. Der Aufbau der proximalen Aufnahme gemäß der dritten Ausführungsform entspricht im Wesentlichen dem der zweiten Ausführungsform. Zusätzlich ist ein dritter proximaler Axialanschlag 74 vorgesehen, der durch eine dritte Scheibe 76 gebildet ist und über eine dritte Feder 78 in eine distale Richtung axialvorgespannt ist. Die drei Scheiben 54, 56, 76 liegen axial aneinander an. Die drei Federn 58, 60, 78 sind radial geschachtelt angeordnet.

Fign. 9 bis 11 zeigen unterschiedliche Ausführungsformen eines distalen Abschnitts des Isolationsmantels 16 und einer distalen Aufnahme des Isolationsmantels 16.

An dem Instrument 2, insbesondere an dem Instrumentenschaft 12 (oder einem damit (translationsfest) verbundenen Bauteil), ist insbesondere ein distaler Axialanschlag 80 zur Begrenzung einer distal gerichteten Axialbewegung (in Richtung zum Werkzeug 4 hin) des Isolationsmantels 16 ausgebildet. Der distale Axialanschlag 80 kann axialfest zu dem Instrumentenschaft 12 aufgenommen sein, insbesondere axialfest mit dem Instrumentenschaft 12 verbunden bzw. an dem Instrumentenschaft 12 ausgebildet sein. In Fig. 9 liegt der Isolationsmantel 16 mit dem ersten (kleineren) Durchmesser, in Fig. 10 liegt der Isolationsmantel 16, dessen Durchmesser sich an seinem distalen Ende von dem zweiten (größeren) Durchmesser auf den ersten (kleineren) Durchmesser verjüngt, und in Fig. 11 liegt der Isolationsmantel 16 mit dem zweiten (größeren) Durchmesser an dem distalen Axialanschlag 80 an.

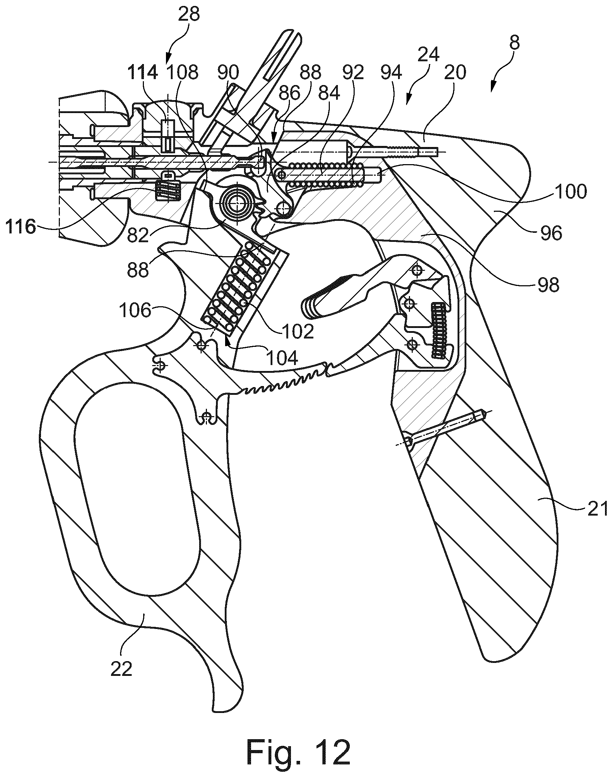

Fig. 12 zeigt eine Längsschnittdarstellung des Handgriffs 8, anhand der die Ausbildung des Getriebes 24 näher erläutert wird. Wie obenstehend beschrieben, ist das Getriebe 24 ausgebildet, um die Schwenkbewegung des Betätigungshebels 22 in die Translationsbewegung der Transmission, insbesondere der Längsbewegung der Zug-Druckstange 14 zu übertragen. Das heißt, dass das Getriebe 24 die (durch die manuelle Betätigung aktuierte) Schwenkbewegung des Betätigungshebels 22 mit der Längsbewegung der Zug-/Druckstange 14 koppelt (und damit (indirekt) mit dem Betätigen des Werkzeugs 4 bzw. der Schwenkbewegung der Werkzeugbranchen 10).

Dabei ist die Betätigungshebelschwenkachse, um die der Betätigungshebel 22 an dem Getriebegehäuse 20 drehbar angelenkt ist, insbesondere zwischen der Translationsachse der Translationsbewegung der Transmission, insbesondere der Schaftachse (d.h. der Längsachse des Instrumentenschafts 12 bzw. der Zug-/Druckstange 14), und einem proximalen Endbereich des Betätigungshebels 22 (d.h. der Betätigungskraft-Angriffsstelle zum Aufbringen der Betätigungskraft) angeordnet. Mit anderen Worten befindet sich die Betätigungshebelschwenkachse (bei Benutzung des Instruments 2 in Vertikalrichtung) unterhalb der Schaftachse.

Gemäß einem Aspekt der vorliegenden Offenbarung ist das Getriebe 24 so ausgebildet ist, dass eine Schwenkbewegung des Betätigungshebels 22 hin zum Griffelement 21 in eine Translationsbewegung der Transmission 14 in Richtung proximal transformiert wird. Das heißt, dass die Schwenkbewegung des Betätigungshebels 22 hin zum Griffelement 21 (das Betätigen des Betätigungshebels 22) in eine proximal gerichtete Längsbewegung der Zug-/Druckstange 14 (eine Zugbewegung der Zug-/Druckstange 14) gewandelt wird.

Insbesondere ist das Getriebe 24 dazu zweiteilig oder mehrteilig ausgebildet. Vorzugsweise bildet das Getriebe 24 einen Kraftübertragungszug (vom Betätigungshebel 22 zur Transmission (Zug-/Druckstange 14)).

Der Kraftübertragungszug weist ein erstes Drehteil (/Stellhebel) 82 auf. Das erste Drehteil 82 steht mit dem Betätigungshebel 22 in Wirkeingriff. Das heißt, dass die Schwenkbewegung des Betätigungshebels 22 mit einer Rotation des erstes Drehteils 82 gekoppelt ist. Das erste Drehteil 82 ist an dem Getriebegehäuse 20 um eine erste Drehachse drehbar angelenkt. Die erste Drehachse ist insbesondere quer bzw. senkrecht zu der Schaftachse. Das erste Drehteil 82 ist insbesondere separat zu dem Betätigungshebel 22 ausgebildet, kann aber alternativ auch an diesem (d.h. an einem Abschnitt des Betätigungshebels 22) ausgebildet sein, auch wenn dies nicht dargestellt ist.

Der Kraftübertragungszug weist ein zweites Drehteil (/Aufnahmeelement/Schließelement) 84 auf. Das zweite Drehteil 84 steht mit dem ersten Drehteil 82 unter Drehrichtungsumkehr in Wirkeingriff, vorzugsweise in Verzahnungseingriff. Das heißt, dass die Rotation des ersten Drehteils 82 mit einer Rotation des zweiten Drehteils 84 gekoppelt ist, und das erste Drehteil 82 und das zweite Drehteil 84 in unterschiedliche Drehrichtungen rotieren. Das zweite Drehteil 84 ist an dem Getriebegehäuse 20 um eine zweite Drehachse drehbar angelenkt. Die zweite Drehachse ist insbesondere quer bzw. senkrecht zu der Schaftachse. Die zweite Drehachse ist vorzugsweise parallel versetzt zu der ersten Drehachse. Das zweite Drehteil 84 hat vorzugsweise einen Kopplungsabschnitt 86 für die Transmission (/ Zug-/Druckstange 14). Der Kopplungsabschnitt 86 ist insbesondere mit der Transmission (/Zug-/Druckstange 14) in Wirkeingriff stehend oder bringbar. Das heißt, dass die Rotation des zweiten Drehteils 84 über den Kopplungsabschnitt 86 mit der Translationsbewegung der Transmission, insbesondere der Längsbewegung der Zug-/Druckstange 14, gekoppelt oder koppelbar ist.

Dazu können das erste Drehteil 82 und das zweite Drehteil 84 miteinander in Verzahnungseingriff stehende Zähne aufweisen. Die Verzahnung kann beispielsweise als eine Evolventenverzahnung ausgebildet sein. Durch den Verzahnungseingriff rotieren das erste Drehteil 82 und das zweite Drehteil 84 in unterschiedliche Drehrichtungen. Dies hat zur Folge, dass die Schwenkbewegung des Betätigungshebels 22 in der Betätigungsrichtung die Längsbewegung der Zug-/Druckstange 14 in die Zugrichtung und die Schwenkbewegung des Betätigungshebels 22 in der Rückstellrichtung die Längsbewegung der Zug-/Druckstange 14 in die Schubrichtung hervorruft (/erzwingt/aktuiert).

Vorzugsweise kann der Instrumentenschaft 12 in gekoppeltem Zustand als Anschlag für die Schwenkbewegung des Betätigungshebels 22, d.h. als Aufschwenkbegrenzung für den Betätigungshebel 22, bzw. als Rotationsbegrenzung für das erste Drehteil 82 und das zweite Drehteil 84 bzw. als Translationsbegrenzung für die Transmission, insbesondere als Längsbegrenzung für die Zug-/Druckstange 14 dienen. Das heißt, dass der Betätigungshebel 22 (nur) innerhalb eines, vorzugsweise beidseitig begrenzten Schwenkbereichs verschwenkbar ist bzw. das erste Drehteil 82 und das zweite Drehteil 84 (nur) innerhalb eines, vorzugsweise beidseitig begrenzten Rotationsbereichs bzw. die Transmission (/Zug-/Druckstange 14) (nur) innerhalb eines, vorzugsweise beidseitig begrenzten Translationsbereichs (/Längsbereich/Arbeitsbereich) längsbeweglich ist, d.h. einen maximalen Hub der Längsbewegung hat. Durch die Kopplung zwischen der Transmission (/Zug-/Druckstange 14) und dem Getriebe 24 (insbesondere dem zweiten Drehteil 82), der Kraftübertragung innerhalb des Getriebes 24 (insbesondere dem zweiten Drehteil 82 und dem ersten Drehteil 82) sowie der Kopplung zwischen dem Getriebe 24 (insbesondere dem ersten Drehteil 82) und dem Betätigungshebel 22 kann der Anschlag am Instrumentenschaft 12 für alle miteinander gekoppelten (oder koppelbaren) Bewegungen dienen.

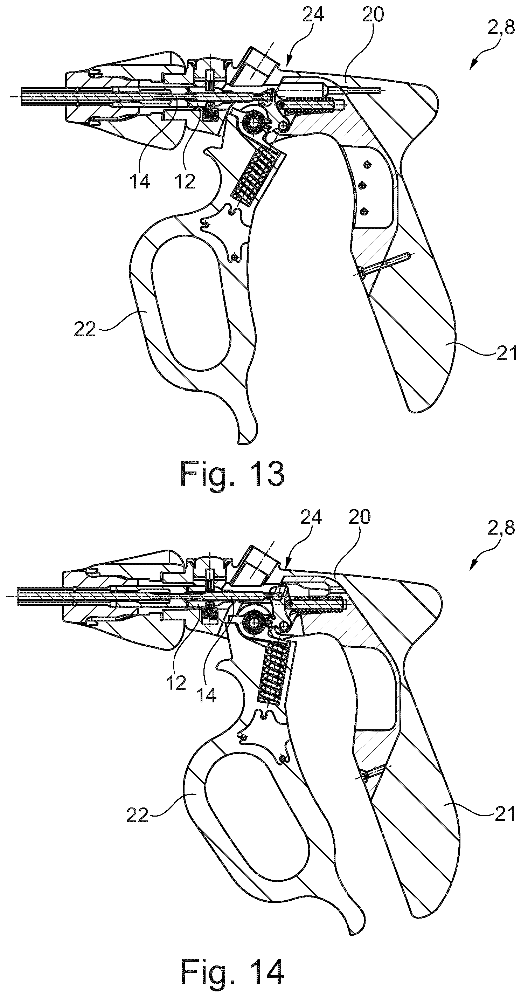

Fign. 13 und 14 zeigen Endstellungen des Translationsbereichs/Arbeitsbereichs der Transmission, in der das Werkzeug 4 vollständig geöffnet bzw. geschlossen ist.

Vorzugsweise kann das Getriebe 24 ein Übersetzungsverhältnis von 1:1 aufweisen. Insbesondere können die Zähne des ersten Drehteils 82 und des zweiten Drehteils 84 auf demselben Durchmesser angeordnet sein. Alternativ könnten die Zähne des ersten Drehteils 82 und des zweiten Drehteils 84 auf unterschiedlichen Durchmessern angeordnet sein, um eine Untersetzung oder eine Übersetzung zu realisieren, auch wenn es nicht dargestellt ist.

Vorzugsweise können die Zähne des ersten Drehteils 82 und/oder die Zähne des zweiten Drehteils 84 (nur) umfangsseitig abschnittsweise, d.h. nicht über einen gesamten Umfang, ausgebildet sein. Dabei kann eine Anzahl der Zähne des ersten Drehteils 82 und/oder des zweiten Drehteils 84, d.h. eine Dimensionierung der umfangsseitig abschnittsweisen Ausbildung, vorzugsweise in Abhängigkeit des maximalen Hubs der Längsbewegung/des begrenzten Längsbereichs der Zug-/Druckstange 14 festgelegt sein. Insbesondere können das erste Drehteil 82 und/oder das zweite Drehteil 84 zwei bis fünf Zähne, vorzugsweise zwei, drei oder vier Zähne, aufweisen. Alternativ könnten die Zähne des ersten Drehteils 82 und/oder des zweiten Drehteils 84 über den gesamten Umfang ausgebildet sein, auch wenn dies nicht dargestellt ist.

Vorzugsweise kann der Kopplungsabschnitt 86 des zweiten Drehteils 84 als eine axial hinterschnittene Ausnehmung 88 ausgebildet sein, in welche die Transmission (Zug-/Druckstange 14) axial hinterschneidend eingreifen kann oder eingreift, um die Drehung des zweiten Drehteils 84 mit der Translationsbewegung (/Längsbewegung der Zug-/Druckstange 14) zu koppeln. Dazu kann die Zug-/Druckstange 14 an ihrem proximalen Ende (Endbereich) eine, insbesondere in Form eines Kugeldruckstücks 90 ausgebildete, radiale (gegenüber einem axial angrenzenden Bereich vergrößerte) Verdickung aufweisen, die in die Ausnehmung 88 des zweiten Drehteils 84 axial hinterschneidend eingreift.

Das Getriebe 24 weist einen an dem zweite Drehteil 84 (drehbar) angelenkten Führungsdorn 92 auf. Der Führungsdorn 92 ist insbesondere an dem zweiten Drehteil 84 um eine Dorndrehachse drehbar angelenkt, so dass das zweite Drehteil 84 und der Führungsdorn 92 relativ zueinander verdreht werden können. Die Dorndrehachse ist insbesondere quer bzw. senkrecht zu der Schaftachse. Vorzugsweise kann die Dorndrehachse parallelversetzt zu der zweiten Drehachse (und/oder der ersten Drehachse) sein. Der Führungsdorn 92 ist längsgeführt, d.h. (nur) entlang seiner Längsachse verschieblich, in dem Getriebegehäuse 20 aufgenommen. Dabei kann die Längsachse des Führungsdorns 92 insbesondere parallelversetzt zu der Schaftachse sein. Vorzugsweise ist der Führungsdorn 92, über eine Feder 94, federvorgespannt in dem Getriebegehäuse 20 aufgenommen. Vorzugsweise kann die Federvorspannung des Führungsdorns 92 der Schwenkbewegung des Betätigungshebels 22 hin zum Griffelement 21 (d.h. der Betätigung) entgegenwirken.

Vorzugsweise kann der Handgriff 8 einen Außengriff 96 und einen fest mit dem Außengriff 96 verbundenen, beispielsweise angeschraubten Innengriff 98 aufweisen. Vorzugsweise kann der Innengriff 98 von dem Außengriff 96 außenseitig abgedeckt sein. Insbesondere kann das zweite Drehteil 84 drehbar an dem Innengriff 98 angelenkt sein. Insbesondere kann der Führungsdorn 92 drehbar an dem Innengriff 98 angelenkt sein. Zudem kann der Führungsdorn 92 in einer Aussparung 100 in dem Innengriff 98 längsgeführt aufgenommen sein.

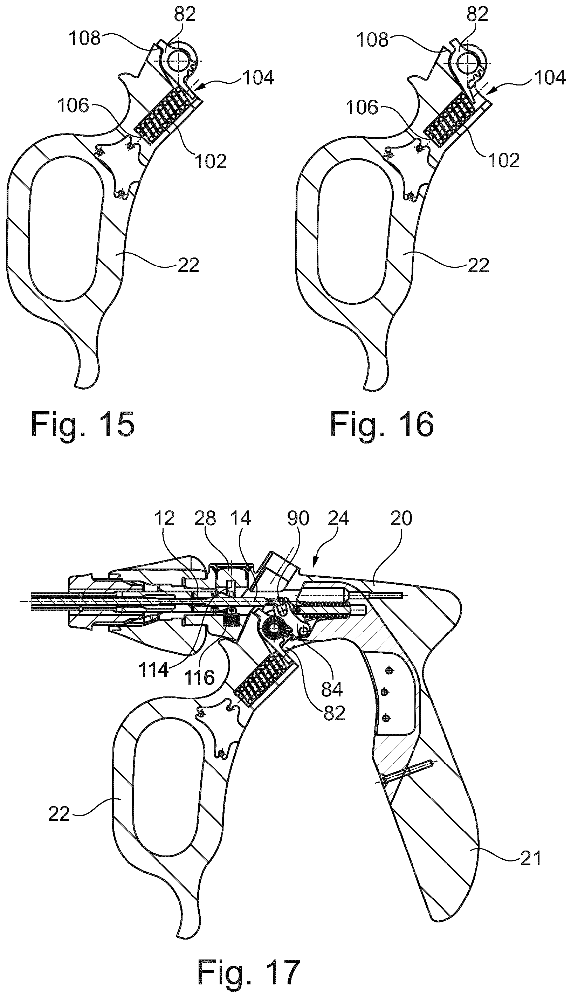

Gemäß einem Aspekt der vorliegenden Offenbarung weist das Getriebe 24 das insbesondere an dem Getriebegehäuse 20 (drehbar) angelenkte erste Drehteil 82 sowie ein die Schwenkbewegung des Betätigungshebels 22 mit einer Rotation des ersten Drehteils 82 koppelndes, elastisches Überlastsicherungselement 102 auf (vgl. insbesondere auch Fign. 15 und 16). Das heißt, dass das Überlastsicherungselement 102 im Kraftfluss/Kraftübertragungszug zwischen dem Betätigungshebel 22 und dem ersten Drehteil 82 angeordnet ist. Das Überlastsicherungselement 102 kann insbesondere als ein Federelement, vorzugsweise als eine Schraubenfeder ausgebildet sein.

Vorzugsweise können der Betätigungshebel 22 und das erste Drehteil 82 um dieselbe Achse schwenkbar bzw. drehbar an dem Getriebegehäuse 20 angelenkt sein. Das heißt, dass die Betätigungshebelschwenkachse vorzugsweise der ersten Drehachse entspricht.

Vorzugsweise kann der Betätigungshebel 22 eine Ausnehmung 104 aufweisen, in der das Überlastsicherungselement 102 aufgenommen ist. Insbesondere kann das Überlastsicherungselement 102 vollständig innerhalb der Ausnehmung 104 angeordnet sein. Das heißt, dass das Überlastsicherungselement 102 vorzugsweise nach außen hin durch den Betätigungshebel 22 abgedeckt ist.

Vorzugsweise kann das Überlastsicherungselement 102 lose in der Ausnehmung 104 aufgenommen sein, d.h. nicht fest mit dem Betätigungshebel 22 und/oder dem ersten Drehteil 82 verbunden sein. Insbesondere kann das Überlastsicherungselement 102 längsgeführt, d.h. d.h. (nur) entlang seiner Längsachse verschieblich bzw. elastisch komprimierbar bzw. verbiegbar in der Ausnehmung 104 aufgenommen sein.

Wie oben beschrieben, kann der Betätigungshebel 22 in die Betätigungsrichtung, d.h. in die Richtung von dem Betätigungshebel 22 aus zu dem Griffelement 21 hin, aktuiert etwa durch Schließen einer Hand des Operateurs/Zusammendrücken von Betätigungshebel 22 und Griffelement 21, und in die Rückstellrichtung, d.h. in die Richtung von dem Betätigungshebel 22 aus von dem Griffelement 21 weg, aktuiert etwa durch Öffnen einer Hand des Operateurs/Auseinanderdrücken von Betätigungshebel 22 und Griffelement 21, verschwenkt werden, d.h. betätigt und rückgestellt werden.

Dabei kann das Überlastsicherungselement 102 vorzugsweise so angeordnet sein, dass es (nur/ausschließlich) in der Betätigungsrichtung (und nicht in der Rückstellrichtung) wirkt. Das heißt, dass das Überlastsicherungselement 102 nur die Schwenkbewegung des Betätigungshebels 22 zum Griffelement 21 hin begrenzt/dämpft/vor Überlast sichert/schützt. Insbesondere können eine Betätigungsübertragungsfläche 106 des Betätigungshebels 22 und eine Rückstellübertragungsfläche 108 des Betätigungshebels 22 voneinander getrennt, d.h. an unterschiedlichen Flächen, ausgebildet sein. Das heißt, dass der Betätigungshebel 22 und das erste Drehteil 82 nicht fest miteinander verbunden sind, und eine Kraftübertragung bei dem Betätigen des Betätigungshebels 22 über die über das Überlastsicherungselement 102 mit dem ersten Drehteil 82 gekoppelte Betätigungsübertragungsfläche 106 und eine Kraftübertragung bei dem Rückstellen des Betätigungshebels 22 (direkt) über die an dem ersten Drehteil 82 anliegende gekoppelte Rückstellübertragungsfläche 108 erfolgt.

Zudem kann das Überlastsicherungselement 102 vorzugsweise so angeordnet und dimensioniert sein, dass eine anfängliche (anfänglich im Sinne der Betätigung, d.h. ausgehend von der Schwenkbewegung aus einer unbetätigten Stellung des Betätigungshebels 22) Kraftübertragung über das Überlastsicherungselement 102 zwischen dem Betätigungshebel 22 und dem ersten Drehteil 82 ein im Wesentlichen lineares Übertragungsverhalten aufweist. Das heißt, dass die Kopplung über das Überlastsicherungselement 102 bei der anfänglichen Kraftübertragung, insbesondere bei Kraftübertragung innerhalb des "normalen" Arbeitsbereichs (und ohne Widerstand am Werkzeug 4), quasi starr/unelastisch ist.

Insbesondere kann das Überlastsicherungselement 102 vorzugsweise so angeordnet und dimensioniert sein, dass das Überlastsicherungselement erst ab einer über den Betätigungshebel 22 aufgebrachten Betätigungskraft größer 200 N, vorzugsweise größer 300 N, (aber maximal erst ab einer über den Betätigungshebel 22 aufgebrachten Betätigungskraft von 1000 N) komprimiert wird.

Gemäß einem Aspekt der vorliegenden Offenbarung kann der Instrumentenschaft 12 an den Handgriff 8 gekoppelt oder koppelbar sein und in gekoppeltem Zustand als Anschlag für die Schwenkbewegung des Betätigungshebels 22 (und der im Kraftübertragungszug damit gekoppelten Bauteile) dienen. Das heißt, dass der Instrumentenschaft 12 im gekoppelten Zustand als Aufschwenkbegrenzung für den Betätigungshebel 22, sowie als Translationsbegrenzung für die Transmission bzw. Längsbegrenzung für die Zug-/Druckstange 14, und damit als Wegbegrenzung für das Werkzeug 4 dient. Das heißt auch, dass in einem entkoppelten Zustand des Instrumentenschafts 12 die Anschlagsfunktion für die Schwenkbewegung des Betätigungshebels 22 sowie die Translationsbegrenzung für die Transmission bzw. die Längsbegrenzung für die Zug-/Druckstange 14, und damit die Wegbegrenzung für das Werkzeug 4 entfällt, so dass die beweglich geführten oder angelenkten Bestandteile des Kraftübertragungszugs des Getriebes 24 bzw. des Instruments 2 sich frei (bezüglich ihres Freiheitsgrads/ihrer aufgrund einer entsprechenden Lagerung/Aufnahme bzw. Anbringung möglichen Bewegung) bewegen können.

Dabei kann der Instrumentenschaft 12 derart als Anschlag dienen, dass eine Entkopplung der Transmission (/Zug-/Druckstange 14) von dem Getriebe 24 nur im entkoppelten Zustand des Instrumentenschafts 12 vom Handgriff 8 infolge des Wegfalls seiner Anschlagfunktion möglich ist. Das heißt, dass eine Entkopplung der Transmission (/Zug-/Druckstange 14) von dem Getriebe 24 im am Handgriff 8 gekoppelten Zustand des Instrumentenschafts 12 infolge dessen Anschlagsfunktion gesperrt ist.

Wie oben beschrieben, kann der durch den Instrumentenschaft 12 gebildete Anschlag vorzugsweise als ein die Translationsbewegung der Transmission (/die Längsbewegung der Zug-/Druckstange 14) insbesondere beidseitig begrenzender Axialanschlag ausgebildet sein.

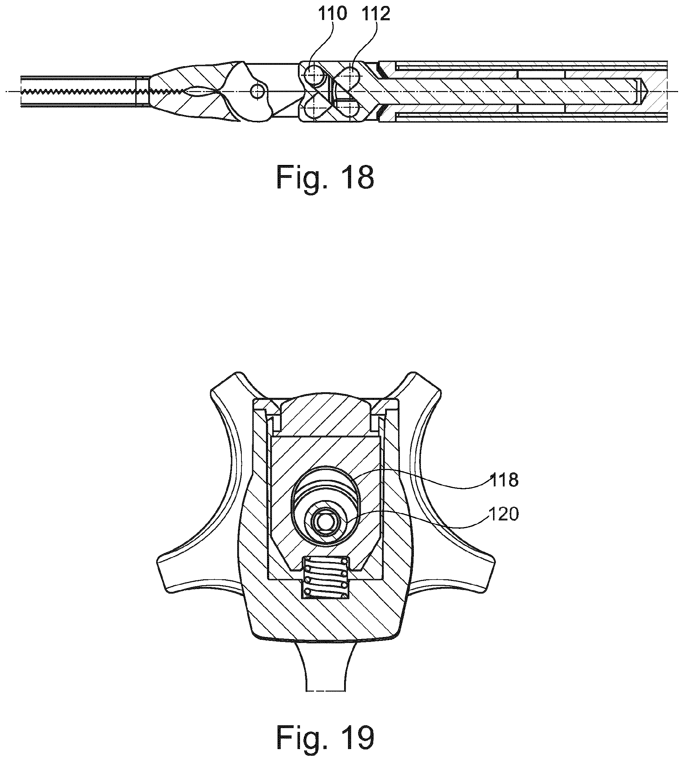

Beispielsweise kann der Anschlag dadurch gebildet sein, dass die Transmission (/Zug-/Druckstange 14) einen Zapfen 110 hat, der in einer Kulisse 112 an dem Instrumentenschaft 12 aufgenommen ist, so dass bei am Kulissenrand anliegendem Zapfen 110 eine weitere Translationsbewegung (in Richtung des Kulissenrands) unterbunden ist (vgl. Fig. 18). Vorzugsweise kann der Anschlag an einem distalen Endbereich des Instrumentenschaft 12 ausgebildet sein.

Vorzugsweise kann das Getriebe 24 das mit dem Betätigungshebel 22 in Wirkverbindung stehende zweite Drehteil 84 aufweisen, dessen Rotation über eine (erste) axiale Formschlussverbindung mit der Translationsbewegung der Transmission (/der Längsbewegung der Zug-/Druckstange 14) koppelbar oder gekoppelt ist. Die erste axiale Formschlussverbindung ist insbesondere durch die axial hinterschnittene Ausnehmung 88 im zweiten Drehteil 84 ausgebildet, in die die Transmission, insbesondere das Kugeldruckstück 90 der Zug-/Druckstange 14 (im gekoppelten Zustand des Instrumentenschafts 12 und damit der Zug-/Druckstange 14) axial hinterschneidend eingreift.

Vorzugsweise kann die Transmission (/Zug-Druckstange 14) im entkoppelten Zustand des Instrumentenschafts 12 so weit translatorisch verschiebbar, insbesondere längsbeweglich, sein und (aufgrund der Kopplung zwischen der Transmission und dem zweiten Drehteil 84 bzw. dem Getriebe 24) kann insbesondere das zweite Drehteil 84 im entkoppelten Zustand des Instrumentenschafts (12) so weit drehbar sein, dass die axiale Formschlussverbindung zwischen der Transmission und dem zweiten Drehteil 84 lösbar ist. Diese Stellung des zweiten Drehteils 84 wird im Folgenden auch als eine Demontagestellung des zweiten Drehteils 84 bezeichnet bzw. als Ladeposition des Instruments 2 bezeichnet (vgl. insbesondere Fig. 17). Insbesondere kann die Transmission (/Zug-/Druckstange 14) (zusammen mit dem Instrumentenschaft 12 bzw. der gesamten Schaftbaugruppe 6) in die distale Richtung (aus dem Handgriff 8 heraus) gezogen werden.

Zudem kann das zweite Drehteil 84 federvorgespannt in dem Handgriff 8, insbesondere dem Getriebegehäuse 20, aufgenommen sein. Dabei kann insbesondere eine Federvorspannung des zweiten Drehteils 84 das zweite Drehteil 84 in die Demontagestellung drücken, in der die erste axiale Formschlussverbindung zwischen der Transmission (/Zug-/Druckstange 14) und dem zweiten Drehteil 84 lösbar ist. Die Federvorspannung kann insbesondere über die den Führungsdorn 92 vorspannende Feder 94 realisiert sein. Die Demontagestellung kann insbesondere einer unbetätigt überstreckten Stellung des Betätigungshebels 22 entsprechen, d.h. einer Stellung, in der der Betätigungshebel 22 weiter von dem Griffelement 21 weg positioniert ist als in der unbetätigten Stellung.

Insbesondere kann der Instrumentenschaft 12 im gekoppelten Zustand axialfest mit dem Handgriff 8 gekoppelt sein und im entkoppelten Zustand axialverschieblich zu dem Handgriff 8 sein. Das heißt, dass eine Kopplung zwischen dem Instrumentenschaft 12 und dem Handgriff 8 insbesondere einer axialfesten Verbindung entspricht.

Wie oben beschrieben kann das Instrument 2 bzw. der Handgriff 8 vorzugsweise den Demontageknopf 28 aufweisen, bei dessen Betätigung (/Drücken) die Schaftbaugruppe 6/der Instrumentenschaft 12 und der Handgriff 8 demontierbar sind, d.h. die Schaftbaugruppe 6/der Instrumentenschaft 12 aus dem Handgriff 8 auskoppelbar ist. Der Demontageknopf 28 kann insbesondere manuell betätigbar sein. Durch ein Betätigen des Demontageknopfs 28 kann der Instrumentenschaft 12 von dem Handgriff 8 axial entkoppelbar sein. Das heißt, dass der Demontageknopf 28 eine Axialverschiebung des Instrumentenschafts 12 relativ zum Handgriff 8 sperren kann. Mit anderen Worten können der Instrumentenschaft 12 und der Handgriff bei betätigtem Demontageknopf 28 zueinander frei axial verschoben werden und bei unbetätigtem Demontageknopf 28 axial miteinander verbunden werden bzw. sein.

Vorzugsweise kann der Demontageknopf 28 einen (längs-)verschieblich in dem Handgriff 8 aufgenommenen Schließschieber 114 aufweisen, dessen Längsachse insbesondere einer Radialrichtung des Instrumentenschafts 12 entspricht. Das heißt, dass der Schließschieber 114 quer bzw. senkrecht zu der Schaftachse verschieblich ist. Der Schließschieber 114 kann vorzugsweise über eine zweite axiale Formschlussverbindung mit dem Instrumentenschaft 12 koppelbar oder gekoppelt sein. Dabei kann die zweite axiale Formschlussverbindung vorzugsweise durch eine (Längs-)Verschiebung des Demontageknopfs 28 (relativ zu dem Handgriff 8) lösbar sein.

Insbesondere kann der Schließschieber 114 federvorgespannt in dem Handgriff aufgenommen sein. Dabei kann die (zweite) axiale Formschlussverbindung vorzugsweise entgegen einer Federvorspannung des Schließschiebers 114 lösbar sein. Das heißt, dass eine Federvorspannung einer Feder 116 den Schließschieber 114 in eine unbetätigte Position drückt bzw. in axial formschlüssigen Eingriff mit dem Instrumentenschaft 12 drückt.

Beispielsweise kann die zweite axiale Formschlussverbindung durch eine in dem Schließschieber 114 ausgebildete langlochartige Kulisse 118 gebildet sein, mit welcher der Instrumentenschaft 12 (bei betätigtem Demontageknopf 28) außer axial formschlüssigem Eingriff ist und somit axial durch die Kulisse 118 hindurchführbar ist und (bei unbetätigtem Demontageknopf 28) in axial formschlüssigen Eingriff ist und somit nicht axial zu der Kulisse 118 (und damit zum Handgriff 8) bewegbar ist. Dazu kann der Instrumentenschaft 12 vorzugsweise eine (etwa umfangsseitig umlaufende) Radialnut 120 aufweisen, in die ein Endbereich/Kulissenrand der Kulisse 118 in der unbetätigten Position des Demontageknopfs 28 eingreift (und dadurch eine Axialbewegung des Instrumentenschafts 12 (in Richtung durch die Kulisse hindurch) unterbindet) und in einer betätigten Position (/längsverschobenen Position) des Demontageknopfs 28 in einem zentralen Bereich/einer Kulissenmitte der Kulisse 118 aufgenommen ist (vgl. Fig. 19).

## Patentansprüche

1. Chirurgischer Pistolen-Handgriff (8) eines oder für ein chirurgisches Instrument (2), insbesondere elektrochirurgisches Instrument (2) der minimalinvasiven Schaftbauart, mit
- einem Getriebegehäuse (20), das sich von distal in Richtung proximal erstreckt;
- einem feststehenden Griffelement (21), das sich in einem Winkel zur distal-proximal-Richtung erstreckt und an einem proximalen Endabschnitt des Getriebegehäuses (an diesem) ausgebildet oder fixiert ist;
- einem am Getriebegehäuse (20) angelenkten, insbesondere manuell betätigbaren, vorzugsweise fingerführbaren Betätigungshebel (22); und
- einem im Getriebegehäuse (20) untergebrachten Getriebe (24), das ausgebildet ist, um eine Schwenkbewegung des Betätigungshebels (22) in eine Translationsbewegung, vorzugsweise Längsbewegung einer Transmission (14), vorzugsweise einer innerhalb eines an den Pistolen-Handgriff (8) distal angekuppelten oder ankuppelbaren Instrumentenschafts (12) gelagerten Zug-/Druckstange (14) zu übertragen,
wobei das Getriebe (24) so ausgebildet ist, dass eine Schwenkbewegung des Betätigungshebels (22) hin zum Griffelement (21) in eine Translationsbewegung der Transmission (14) in Richtung proximal, insbesondere eine proximal gerichtete Längsbewegung der Zug-/Druckstange (14), transformiert wird,
**dadurch gekennzeichnet, dass**
das Getriebe (24) einen Kraftübertragungszug bildet aufweisend ein erstes, mit dem Betätigungshebel (22) in Wirkeingriff stehendes Drehteil (82) und ein zweites Drehteil (84), das mit dem ersten Drehteil (82) unter Drehrichtungsumkehr in Wirkeingriff, vorzugsweise Verzahnungseingriff steht und das einen Kopplungsabschnitt (86) für die Transmission (14) hat,
wobei das Getriebe (24) einen an dem zweiten Drehteil (84) angelenkten Führungsdorn (92) aufweist, der in dem Getriebegehäuse (20) längsgeführt aufgenommen ist.

2. Pistolen-Handgriff (8) nach Anspruch 1, **dadurch gekennzeichnet, dass** der Führungsdorn (92) in dem Getriebegehäuse (20) parallel zu der Translationsachse, längsgeführt aufgenommen ist.

3. Pistolen-Handgriff (8) nach Anspruch 1 oder 2, **dadurch gekennzeichnet, dass** der Führungsdorn (92) in dem Getriebegehäuse (20) federvorgespannt aufgenommen ist.

4. Pistolen-Handgriff (8) nach Anspruch 3, **dadurch gekennzeichnet, dass** eine Federvorspannung des Führungsdorns (92) der Schwenkbewegung des Betätigungshebels (22) hin zum Griffelement (21) entgegenwirkt.

5. Pistolen-Handgriff (8) nach einem der Ansprüche 1 bis 4, **dadurch gekennzeichnet, dass** der Betätigungshebel (22) um eine Betätigungsschwenkachse schwenkbar an dem Getriebegehäuse (20) gelagert ist und die Betätigungsschwenkachse zwischen einem proximalen Endbereich des Betätigungshebels (22) zum Aufbringen einer Betätigungskraft und einer Translationsachse der Translationsbewegung der Transmission, insbesondere einer Längsachse der Zug-/Druckstange (14) angeordnet ist.

6. Pistolen-Handgriff (8) nach einem der Ansprüche 1 bis 5, **dadurch gekennzeichnet, dass** das erste Drehteil (82) und das zweite Drehteil (84) miteinander in Wirkeingriff, vorzugsweise Verzahnungseingriff stehende, umfangsseitig abschnittsweise ausgebildete Zähne aufweisen.

7. Pistolen-Handgriff (8) nach Anspruch 6, **dadurch gekennzeichnet, dass** eine Anzahl der Zähne des ersten Drehteils (82) und/oder des zweiten Drehteils (84) in Abhängigkeit eines maximalen Hubs der Translationsbewegung der Transmission (14) festgelegt ist.

8. Pistolen-Handgriff (8) nach einem der Ansprüche 1 bis 7, **dadurch gekennzeichnet, dass** der Kopplungsabschnitt (86) des zweiten Drehteils als eine axial hinterschnittene Ausnehmung (88) zur axial hinterschneidend eingreifenden Aufnahme der Transmisson (14) ausgebildet ist, um eine Drehung des zweiten Drehteils (84) mit der Translationsbewegung zu koppeln.

9. Pistolen-Handgriff (8) nach einem der Ansprüche 1 bis 8, **dadurch gekennzeichnet, dass** das feststehende Griffelement (21) proximal zu dem Betätigungshebel (22) angeordnet ist.

10. Pistolen-Handgriff (8) nach einem der Ansprüche 1 bis 9, **dadurch gekennzeichnet, dass** das feststehende Griffelement (21) grifföffnungsfrei, insbesondere ohne eine Fingeraufnahmeöffnung und/oder ohne eine Daumenaufnahmeöffnung, ausgebildet ist.

11. Pistolen-Handgriff (8) nach einem der Ansprüche 1 bis 10, **dadurch gekennzeichnet, dass** das Getriebe ein Übersetzungsverhältnis von 1:1 hat.

12. Chirurgisches Instrument (2), insbesondere elektrochirurgisches Instrument (2) der minimalinvasiven Schaftbauart, mit einem Pistolen-Handgriff (8) nach einem der Ansprüche 1 bis 11.

13. Instrument (2) nach Anspruch 12, mit einem Instrumentenschaft (12), der distal an dem Pistolen-Handgriff (8) angekuppelt oder ankuppelbar ist, **dadurch gekennzeichnet, dass** der Instrumentenschaft (12) in gekoppeltem Zustand als Anschlag für die Schwenkbewegung des Betätigungshebels (22) dient.

## Claims

1. A surgical pistol-grip handle (8) of or for a surgical instrument (2), in particular an electrosurgical instrument (2) of the minimally invasive shaft design, comprising
- a gearing housing (20) extending from the distal in the proximal direction;
- a stationary handle element (21) extending at an angle to the distal-proximal direction and configured at or fixed to a proximal end portion of the gearing housing (on this);
- an actuating lever (22) hinged to the gearing housing (20), in particular being manually actuatable, preferably finger-guidable; and
- a gearing (24) housed in the gearing housing (20) and configured to transmit a pivoting movement of the actuating lever (22) into a translational movement, preferably a longitudinal movement, of a transmission (14), preferably of a pull/push rod (14) mounted within an instrument shaft (12) distally coupled or coupleable to the pistol-grip handle (8),
wherein
the gearing (24) is configured such that a pivoting movement of the actuating lever (22) toward the handle element (21) is transformed into a translational movement of the transmission (14) in the proximal direction, in particular a proximally directed longitudinal movement of the pull/push rod (14),
**characterized in that** the gearing (24) forms a power transmission train comprising a first rotating part (82) in operating engagement with the actuating lever (22), and a second rotating part (84) which is in operating engagement, preferably meshing engagement, with the first rotating part (82) under reversal of the direction of rotation and which has a coupling portion (86) for the transmission (14),
wherein the gearing (24) has a guiding spike (92) hinged on the second rotating part (84) and housed longitudinally guided in the gearing housing (20).

2. The pistol-grip handle (8) according to claim 1, **characterized in that** the guiding spike (92) is housed longitudinally guided in the gearing housing (20) parallel to the translational axis.

3. The pistol-grip handle (8) according to claim 1 or 2, **characterized in that** the guiding spike (92) is housed in the gearing housing (20) in a spring-pretensioned manner.

4. The pistol-grip handle (8) according to claim 3, **characterized in that** a spring pretension of the guiding spike (92) counteracts the pivoting movement of the actuating lever (22) toward the handle element (21).

5. The pistol-grip handle (8) according to one of claims 1 to 4, **characterized in that** the actuating lever (22) is mounted on the gearing housing (20) so as to be pivotable about an actuating pivot axis, and the actuating pivot axis is arranged between a proximal end region of the actuating lever (22) for applying an actuating force and a translational axis of the translational movement of the transmission, in particular a longitudinal axis of the pull/push rod (14).

6. The pistol-grip handle (8) according to one of claims 1 to 5, **characterized in that** the first rotating part (82) and the second rotating part (84) have teeth configured in sections on their circumference, which are in operating engagement, preferably meshing engagement, with each other.

7. The pistol-grip handle (8) according to claim 6, **characterized in that** a number of the teeth of the first rotating part (82) and/or of the second rotating part (84) is determined as a function of a maximum stroke of the translational movement of the transmission (14).

8. The pistol-grip handle (8) according to one of claims 1 to 7, **characterized in that** the coupling portion (86) of the second rotating part is configured as an axial undercut recess (88) for axially undercut, engaging receiving of the transmission (14) in order to couple the rotation of the second rotating part (84) with the translational movement.

9. The pistol-grip handle (8) according to one of claims 1 to 8, **characterized in that** the stationary handle element (21) is arranged proximally to the actuating lever (22).

10. The pistol-grip handle (8) according to one of claims 1 to 9, **characterized in that** the stationary handle element (21) is configured without a grip opening, in particular without a finger receptacle opening and/or without a thumb receptacle opening.

11. The pistol-grip handle (8) according to one of claims 1 to 10, **characterized in that** the gearing has a transmission ratio of 1:1.

12. A surgical instrument (2), in particular electrosurgical instrument (2) of the minimally invasive shaft design, with a pistol-grip handle (8) according to one of claims 1 to 11.

13. The instrument (2) according to claim 12, with an instrument shaft (12) that is coupled or coupleable distally to the pistol-grip handle (8), **characterized in that** the instrument shaft (12) serves as a stop for the pivoting movement of the actuating lever (22) in the coupled state.

## Revendications

1. Poignée en forme de pistolet chirurgical (8) d'un ou pour un instrument (2) chirurgical, en particulier un instrument électrochirurgical (2) avec une conception d'arbre mini-invasive, avec
- un boîtier d'engrenage (20) qui s'étend de la direction distale à la direction proximale ;
- un élément de préhension (21) fixe qui s'étend selon un angle par rapport à la direction distale-proximale et est conçu ou fixé à une section d'extrémité proximale du boîtier d'engrenage (à celui-ci) ;
- un levier d'actionnement (22) articulé au boîtier d'engrenage (20), en particulier actionnable manuellement, de préférence pouvant être guidé au doigt ; et
- un engrenage (24) logé dans le boîtier d'engrenage (20) qui est conçu afin de transférer un mouvement de pivotement du levier d'actionnement (22) en un mouvement de translation, de préférence un mouvement longitudinal d'une transmission (14), de préférence d'une tige de traction/poussée (14) logée à l'intérieur d'un arbre d'instrument (12) couplé ou pouvant être couplé distalement à la poignée en forme de pistolet (8),
dans lequel l'engrenage (24) est conçu de sorte qu'un mouvement de pivotement du levier d'actionnement (22) vers l'élément de préhension (21) soit transformé en un mouvement de translation de la transmission (14) dans la direction proximale, en particulier un mouvement longitudinal dirigé dans la direction proximale de la tige de traction/poussée (14),
**caractérisé en ce que**
l'engrenage (24) forme un train de transmission de force présentant une première partie rotative (82) se trouvant en prise active avec le levier d'actionnement (22) et une seconde partie rotative (84) qui se trouve en prise active avec la première partie rotative (82) en inversant le sens de rotation, de préférence en prise par enclenchement et qui présente une section de couplage (86) pour la transmission (14),
dans lequel l'engrenage (24) présente une épine de guidage (92) articulée à la seconde partie rotative (84) qui est logée en étant guidée longitudinalement dans le boîtier d'engrenage (20).

2. Poignée en forme de pistolet (8) selon la revendication 1, **caractérisée en ce que** l'épine de guidage (92) est logée en étant guidée longitudinalement dans le boîtier d'engrenage (20) parallèlement à l'axe de translation.

3. Poignée en forme de pistolet (8) selon la revendication 1 ou 2, **caractérisée en ce que** l'épine de guidage (92) est logée en étant précontrainte par ressort dans le boîtier d'engrenage (20).

4. Poignée en forme de pistolet (8) selon la revendication 3, **caractérisée en ce qu'**une précontrainte par ressort de l'épine de guidage (92) agit à l'encontre du mouvement de pivotement du levier d'actionnement (22) vers l'élément de préhension (21).

5. Poignée en forme de pistolet (8) selon l'une quelconque des revendications 1 à 4, **caractérisée en ce que** le levier d'actionnement (22) est logé de manière pivotante autour d'un axe de pivotement d'actionnement au niveau du boîtier d'engrenage (20), et l'axe de pivotement d'actionnement est agencé entre une zone d'extrémité proximale du levier d'actionnement (22) pour appliquer une force d'actionnement et un axe de translation du mouvement de translation de la transmission, en particulier un axe longitudinal de la tige de traction/poussée (14).

6. Poignée en forme de pistolet (8) selon l'une quelconque des revendications 1 à 5, **caractérisée en ce que** la première partie rotative (82) et la seconde partie rotative (84) présentent des dents conçues par sections sur la périphérie, qui se trouvent en prise active entre elles, de préférence en prise par enclenchement.

7. Poignée en forme de pistolet (8) selon la revendication 6, **caractérisée en ce qu'**un nombre de dents de la première partie rotative (82) et/ou de la seconde partie rotative (84) est défini en fonction d'une course maximale du mouvement de translation de la transmission (14).

8. Poignée en forme de pistolet (8) selon l'une quelconque des revendications 1 à 7, **caractérisée en ce que** la section de couplage (86) de la seconde partie rotative est conçue comme un évidement (88) contre-dépouillé axialement pour le logement de la transmission (14) s'engageant par contre-dépouille axiale afin de coupler une rotation de la seconde partie rotative (84) avec le mouvement de translation.

9. Poignée en forme de pistolet (8) selon l'une quelconque des revendications 1 à 8, **caractérisée en ce que** l'élément de préhension (21) fixe est agencé de manière proximale par rapport au levier d'actionnement (22).

10. Poignée en forme de pistolet (8) selon l'une quelconque des revendications 1 à 9, **caractérisée en ce que** l'élément de préhension (21) fixe est conçu sans ouverture de préhension, en particulier sans une ouverture de logement de doigt et/ou sans une ouverture de logement de pouce.

11. Poignée en forme de pistolet (8) selon l'une quelconque des revendications 1 à 10, **caractérisée en ce que** l'engrenage présente un rapport de transmission de 1:1.

12. Instrument chirurgical (2), en particulier instrument électrochirurgical (2) avec une conception d'arbre mini-invasive, avec une poignée en forme de pistolet (8) selon l'une quelconque des revendications 1 à 11.

13. Instrument (2) selon la revendication 12, avec un arbre d'instrument (12) qui est ou peut être couplé distalement à la poignée en forme de pistolet (8), **caractérisé en ce que** l'arbre d'instrument (12) dans l'état couplé sert de butée pour le mouvement de pivotement du levier d'actionnement (22).
